(19) Europäisches Patentamt
European Patent Office
Office européen des brevets

(11) EP 1 273 658 A1

(12) EUROPEAN PATENT APPLICATION
published in accordance with Art. 158(3) EPC

(43) Date of publication:
08.01.2003 Bulletin 2003/02

(21) Application number: 01917583.5

(22) Date of filing: 29.03.2001

(51) Int Cl.[7]: **C12N 15/12**, C12N 1/21, C07K 14/47, C07K 16/18, C12P 21/02, G01N 33/53, A61K 38/00, A61K 45/00, A61K 48/00, A61P 35/04

(86) International application number:
PCT/JP01/02615

(87) International publication number:
WO 01/075104 (11.10.2001 Gazette 2001/41)

(84) Designated Contracting States:
AT BE CH CY DE DK ES FI FR GB GR IE IT LI LU MC NL PT SE TR
Designated Extension States:
AL LT LV MK RO SI

(30) Priority: 30.03.2000 JP 2000093575

(71) Applicant: Takeda Chemical Industries, Ltd.
Osaka-shi, Osaka 541-0045 (JP)

(72) Inventors:
- OHTAKI, Tetsuya
Tsukuba-shi, Ibaraki 305-0821 (JP)
- SHINTANI, Yasushi
Tsukuba-shi, Ibaraki 305-0821 (JP)
- TERAO, Yasuko
Tsukuba-shi, Ibaraki 305-0034 (JP)
- KUMANO, Satoshi
Tsukuba-shi, Ibaraki 305-0035 (JP)

(74) Representative: Keller, Günter, Dr. et al
Lederer & Keller
Patentanwälte
Prinzregentenstrasse 16
80538 München (DE)

# (54) NOVEL PROTEIN, DNA THEREOF AND PROCESS FOR PRODUCING THE SAME

(57) The present invention relates to a novel protein containing a peptide having a ligand activity to a G-protein copuled receptor protein; amides, esters, salts of the peptides; and drugs containing the same.



Printed by Jouve, 75001 PARIS (FR)

## Description

### TECHNICAL FIELD OF THE INVENTION

The present invention relates to a protein comprising a peptide which has a ligand activity to a novel G protein-coupled receptor protein derived from rat brain stem and its peripheral region and human brain, and a amide or ester of the peptide, or a salt thereof.

### BACKGROUND ART

Physiological active substances such as various hormones and neurotransmitters regulate the biological function via specific receptor proteins present on cell membranes. Many of these receptor proteins are coupled with guanine nucleotide-binding protein (hereinafter sometimes simply referred to as G protein) and mediate the intracellular signal transduction via activation of G protein. These receptor proteins possess the common structure containing seven transmembrane domains and are thus collectively referred to as G protein-coupled receptors or seven-transmembrane receptors (7TMR).

G protein-coupled receptor proteins present on the cell surface of each functional cell and organ in the body, and play important physiological roles as the target of the molecules that regulate the functions of the cells and organs, e.g., hormones, neurotransmitters, physiologically active substances and the like. Receptors transmit signals to cells via binding with physiologically active substances, and the signals induce various reactions such as activation and inhibition of the cells.

To clarify the relationship between substances that regulate complex biological functions in various cells and organs, and their specific receptor proteins, in particular, G protein-coupled receptor proteins, would elucidate the functional mechanisms in various cells and organs in the body to provide a very important means for development of drugs closely associated with the functions.

For example, in various organs, their physiological functions are controlled *in vivo* through regulation by many hormones, hormone-like substances, neurotransmitters or physiologically active substances. In particular, physiologically active substances are found in numerous sites of the body and regulate the physiological functions through their corresponding receptor proteins. However, it is supposed that many unknown hormones, neurotransmitters or many other physiologically active substances still exist in the body and, as to their receptor proteins, many of these proteins have not yet been reported. In addition, it is still unknown if there are subtypes of known receptor proteins.

It is very important for development of drugs to clarify the relationship between substances that regulate elaborated functions *in vivo* and their specific receptor proteins. Furthermore, for efficient screening of agonists and antagonists to receptor proteins in development of drugs, it is required to clarify functional mechanisms of receptor protein genes expressed *in vivo* and express the genes in an appropriate expression system.

In recent years, random analysis of cDNA sequences has been actively studied as a means for analyzing genes expressed *in vivo.* The sequences of cDNA fragments thus obtained have been registered on and published to databases as Expressed Sequence Tag (EST). However, since many ESTs contain sequence information only, it is difficult to predict their functions from the information.

Substances which inhibit the binding between G protein-coupled proteins and physiologically active substances (i.e. ligands) and substances which bind and induce signals similar to those induced by physiologically active substances (i.e. ligands) have been used as antagonists and agonists specific to the receptors, or pharmaceuticals which regulate the biological functions. Therefore, discovering a novel G protein-coupled receptor which can be targeted for pharmaceutical development, and a ligand specific to the receptor are very important ways for searching for an agonist and antagonist of the receptor.

Furthermore, elucidating the physiological function of receptors and predicting the role of receptors in a human, which are carried out by obtaining a counterpart of a human gene encoding the receptor or ligand from a rodent (rat, mouse), chimpanzee, monkey, etc.; by investigating the chemical properties and biological activities of these gene products; and by examining precisely their qualitative and quantitative profiles inside an animal body or physiological mechanisms, are very important for creating an effective pharmaceutical.

In addition, for selecting candidate compounds for an agonist or antagonist, considering species difference is an essential step for drug development.

### SUMMARY OF THE INVENTION

The present invention provides, as described above, rat and mouse homologues of a human ligand protein specific to the human useful novel G-protein coupled receptor protein (hereinafter, which is referred to as "the ligand protein of the present invention").

Until now, the inventors searched for a peptide which binds to the G-protein coupled receptor derived from rat brain stem and human brain and has activity of inducing increase in intracellular Ca ion concentration. As a result, the inventors found that a C-terminal peptide of a protein encoded by a tumor-metastasis suppressor gene, KiSS-1 (Genomics, vol.54, 145-148, 1998) activated the receptor, and confirmed that the peptide consisting of 54 amino acids of KiSS-1, and a C-terminal partial peptide thereof has the ligand activity (JP-A 2000-312590).

It is expected that a peptide generated through cleavage of the KiSS-1 gene product have activity of inhibiting tumor metastasis, because the gene is the tumor-metastasis suppressor gene. In addition, considering that the gene is expressed in a large amount in placenta, and that the human G-protein coupled receptor protein, hOT7T175 (in which h means human) is expressed in large quantity in placenta, it is predicted that the peptide plays an important role in placenta. Further, because expression of the receptor is also relatively high in pancreas inside a human body, the peptide may have some physiological function in pancreas. Rat ligand and rat receptor rOT7T175 (in which r means rat) are both highly expressed in cecum and large intestine, indicating some physiological function of the peptide in these tissues. With extensive research, the inventors successfully isolated cDNA encoding a sequence highly homologous to the human KiSS-1 gene from rat liver cDNA using PCR primers prepared on the basis of the sequence of human KiSS-1 gene, and determined its nucleic acid sequence completely. The amino acid sequence deduced from the nucleic acid sequence revealed that the nucleic acid sequence encodes a protein which has significantly high homology to the amino acid sequence of human KiSS-1. The inventors also isolated cDNA encoding a sequence highly homologous to the human KiSS-1 gene from mouse embryo cDNA, and confirmed that its sequence encodes a protein which has significantly high homology to the amino acid sequence of human and rat KiSS-1.

Based on these findings, the present inventors have continued further extensive studies and as a result, have come to accomplish the present invention. Thus, the present invention relates to the following:

(1) a protein comprising the same or substantially the same amino acid sequence as that represented by SEQ ID NO:1 or NO:3, or a salt thereof;
(2) the protein or salt thereof according to the above (1), wherein substantially the same amino acid sequence is one represented by SEQ ID NO:2;
(3) a partial peptide of the protein according to the above (1), which comprises the amino acid sequence of from the 132 to 141th residues from the N terminal of the amino acid sequence represented by SEQ ID NO:1, or a salt thereof;
(4) the partial peptide or salt thereof according to the above (3), which comprises the same or substantially the same amino acid sequence as that of from the 127 to 141th residues from the N terminal of the amino acid sequence represented by SEQ ID NO:1;
(5) the partial peptide or salt thereof according to the above (3), which has the same or substantially the same amino acid sequence as that of from the 90 to 141th residues from the N terminal of the amino acid sequence represented by SEQ ID NO:1;
(6) a partial peptide of the protein according to the above (1), which has the same or substantially the same amino acid sequence as that of from the 94 to 145th residues from the N terminal of the amino acid sequence represented by SEQ ID NO:2, or a salt thereof;
(7) a partial peptide of the protein according to the above (1), which comprises the amino acid sequence of from the 110 to 119th residues from the N terminal of the amino acid sequence represented by SEQ ID NO:3, or a salt thereof;
(8) the partial peptide or salt thereof according to the above (7), which comprises the same or substantially the same amino acid sequence as that of from the 105 to 119th residues from the N terminal of the amino acid sequence represented by SEQ ID NO:3;
(9) the partial peptide or salt thereof according to the above (7), which has the same or substantially the same amino acid sequence as that of from the 68 to 119th residues from the N terminal of the amino acid sequence represented by SEQ ID NO:3;
(10) a polynucleotide comprising a polynucleotide encoding the protein according to the above (1);
(11) a polynucleotide comprising a polynucleotide encoding the partial peptide according to the above (3), (6) or (7),
(12) the polynucleotide according to the above (10) or (11), which is DNA;
(13) the polynucleotide according to the above (10), which has the nucleic acid sequence represented by SEQ ID NO:4, NO:5 or NO:6;
(14) a recombinant vector comprising the polynucleotide according to the above (10) or (11);
(15) a transformant transformed with the recombinant vector according to the above (14);
(16) a method of producing the protein or salt thereof according to the above (1), or the partial peptide or salt thereof according to the above (3), (6) or (7), which comprises culturing the transformant according to (15), and making it produce and accumulate the protein according to the above (1), or the partial peptide according to the above (3), (6) or (7);

(17) an antibody to the protein or salt thereof according to the above (1), or the partial peptide or salt thereof according to the above (3), (6) or (7);

(18) the antibody according to (17), which is a neutralizing antibody capable of inactivating signal transduction of the protein according to (1) or the partial peptide according to the above (3), (6) or (7);

(19) a method of screening a compound or salt thereof which alters the binding property between a receptor and the protein or salt thereof according to the above (1) or the partial peptide or salt thereof according to the above (3), (6) or (7), which comprises using the protein or salt thereof according to the above (1) or the partial peptide or salt thereof according to the above (3), (6) or (7);

(20) a kit for screening a compound or salt thereof which alters the binding property between a receptor and the protein or salt thereof according to the above (1) or the partial peptide or salt thereof according to the above (3), (6) or (7), which comprises the protein or salt thereof according to the above (1) or the partial peptide or salt thereof according to the above (3), (6) or (7);

(21) the screening method according to the above (19) or the screening kit according to the above (20), wherein the receptor is a protein or salt thereof having the same or substantially the same amino acid sequence as that represented by SEQ ID NO:7, NO:8 or NO:24;

(22) a compound or salt thereof which alters the binding property between a receptor and the protein or salt thereof according to any one of the above (1) and (3) to (5), which is obtainable using the screening method according to the above (19) or the screening kit according to the above (20);

(23) the compound or salt thereof according to the above (22), which is an agonist;

(24) a pharmaceutical composition comprising a compound or salt thereof which alters the binding property between a receptor and the protein or salt thereof according to the above (1) or the partial peptide or salt thereof according to the above (3), (6) or (7), which is obtainable using the screening method according to the above (19) or the screening kit according to the above (20);

(25) the pharmaceutical composition according to the above (24), which is an agent for inhibiting tumor metastasis;

(26) a method of quantifying the protein according to the above (1) or the partial peptide according to the above (3), (6) or (7), which comprises using the antibody according to (17);

(27) a pharmaceutical composition comprising the protein or salt thereof according to the above (1) or the partial peptide or salt thereof according to the above (3), (6) or (7);

(28) the pharmaceutical composition according to the above (27), which is an agent for inhibiting tumor metastasis.

## BRIEF DESCRIPTION OF THE DRAWING

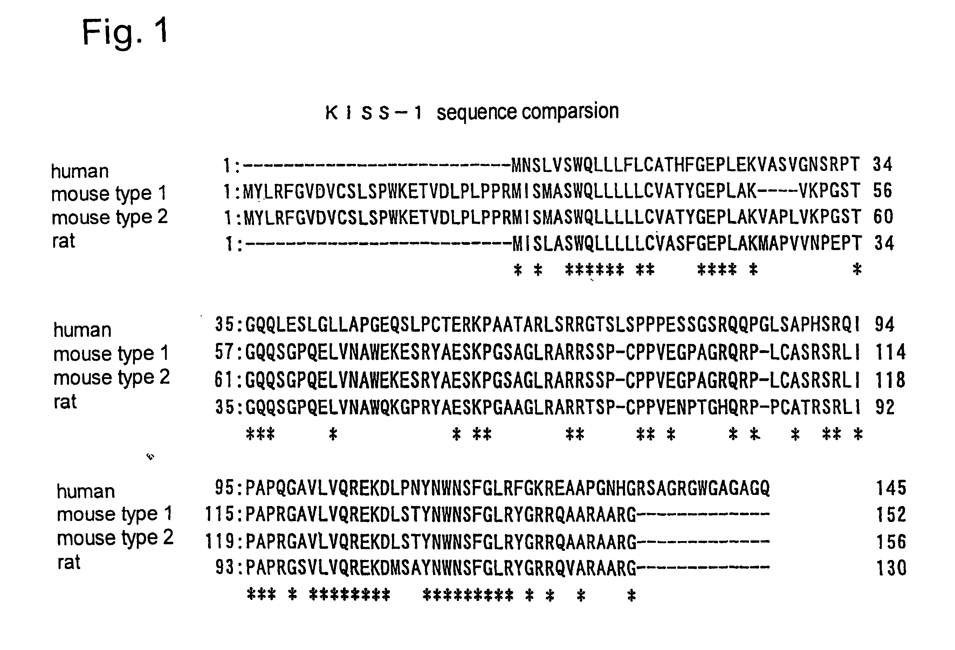

FIG. 1 shows the comparison among human, mouse type 1, mouse type 2, and rat KiSS-1 proteins in terms of amino acid sequence. The identical amino acids among these 4 different homologs are designated by asterisk. Amino acids are represented by one-letter abbreviation.

## BEST MODE FOR CARRYING OUT THE INVENTION

The protein of the present invention includes a human ligand protein specific to the above-mentioned novel human G protein-coupled receptor protein (hOT7T175), rat and mouse homologs thereof, and mature forms thereof.

These proteins may be derived from any cells (e.g. splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g. macrophage, T cells, B cells, natural killer cells, mast cells, neutrophil, basophil, eosinophil, monocyte), megakaryocyte, synovial cells, chondrocytes, bone cells, osteoblasts, osteoclasts, mammary gland cells, hepatocytes or interstitial cells, the corresponding precursor cells, stem cells, cancer cells, etc.), blood cells, or any tissues where such cells are present, e.g. brain or any region of the brain (e.g. olfactory bulb, amygdaloid nucleus, basal ganglia, hippocampus, thalamus, hypothalamus, subthalamic nucleus, cerebral cortex, medulla oblongata, cerebellum, occipital pole, frontal lobe, temporal lobe, putamen, caudate nucleus, corpus callosum, substantia nigra), spinal cord, hypophysis, stomach, pancreas, kidney, liver, gonad, thyroid, gall-bladder, bone marrow, adrenal gland, skin, muscle, lung, gastrointestinal tract (e.g. large intestine and small intestine), blood vessel, heart, thymus, spleen, submandibular gland, peripheral blood, peripheral blood cells, prostate, testis, ovary, placenta, uterus, bone, joint, skeletal muscle, etc. (especially brain or any of brain regions) of human and other mammals (e.g. guinea pigs, rats, mice, rabbits, swine, sheep, bovine, monkeys, etc.). The proteins may be a synthetic protein.

The protein of the present invention has activity of binding to G-protein coupled receptor proteins in a specific manner. The G-protein coupled receptor proteins include OT7T175 derived from a human, OT7T175 derived from a rat, and their homologues derived from other mammals, such as a monkey and mouse. Examples of the protein of the present invention includes the type 1 and type 2 proteins derived from a mouse (comprising the amino acid sequence represented by SEQ ID NO:1 and NO:2, respectively), and the rat protein (comprising the amino acid sequence represented by SEQ ID NO:3), and also mature polypeptides of these proteins. The mouse type 1 mature polypeptide refers to a protein having the amino acid sequence from 90th to 141th residues from the N-terminal of the amino acid sequence represented by SEQ ID NO:1. The mouse type 2 mature polypeptide refers to a protein having the amino acid sequence from 94th to 145th residues from the N-terminal of the amino acid sequence represented by SEQ ID NO:2. The rat mature polypeptide refers to a protein having the amino acid sequence from 68th to 119th residues from the N-terminal of the amino acid sequence represented by SEQ ID NO:3.

In this specification, "substantially the same amino acid sequence" refers to a variant of a reference amino acid sequence wherein (i) at least one or more (preferably approximately 1 to 30, more preferably approximately 1 to 10, most preferably several (1 or 2)) amino acids are deleted from the reference sequence; (ii) at least one or more (preferably approximately 1 to 30, more preferably approximately 1 to 10, most preferably several (1 or 2)) amino acids are added to the reference sequence; (iii) at least one or more (preferably approximately 1 to 30, more preferably approximately 1 to 10, most preferably several (1 or 2)) amino acids of the reference sequence are substituted with other amino acids; or (iv) any combination of these modifications is included in the reference sequence.

Specifically, for example, substantially the same amino acid sequence as that represented by SEQ ID NO:1 or NO:3 includes an amino acid sequence having at least about 70% homology, preferably at least about 80% homology, more preferably at least about 90% homology, and most preferably at least about 95% homology to the amino acid sequence represented by SEQ ID NO:1 or NO:3.

Proteins which comprise substantially the same amino acid sequence as that of the protein of the present invention, and ones which have substantially the same amino acid sequence as that of the protein of the present invention have substantially the same property as that of the protein of the present invention. That is, these proteins have the property of binding to the G protein-coupled receptor proteins in a specific manner. The receptors preferably include OT7T175 derived from a human (SEQ ID NO:7), OT7T175 derived from a rat (SEQ ID NO:8), and OT7T175 derived from a mouse (SEQ ID NO:24). In more details, said property includes the binding activity to the G-protein coupled receptor proteins, the signal transduction activity, and the like. It is preferred that the properties or activities of the above-mentioned proteins are equivalent (e.g., about 0.01- to 100-fold, preferably about 0.5- to 20-fold, more preferably about 0.5- to 2-fold), but some quantitative factors, such as the level of activity, the molecular weight of the protein, may be different. These activities can be measured according to a publicly known method, for example, by the screening method as described below.

In the present specification, the proteins of the present invention are represented in accordance with the conventional way of describing peptides so as to place the N-terminus (amino terminus) on the left side and the C-terminus (carboxyl terminus) on the right side. In the ligand proteins of the present invention, including the ligand protein comprising the amino acid sequence shown by SEQ ID NO:1, the C-terminus is usually in the form of a carboxyl group (-COOH) or a carboxylate ($-COO^-$) but may be in the form of an amide ($-CONH_2$) or an ester (-COOR).

Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; a $C_{7-14}$ aralkyl group such as a phenyl-$C_{1-2}$-alkyl group, e.g., benzyl, phenethyl, etc., or an α-naphthyl-$C_{1-2}$-alkyl group such as α-naphthylmethyl, etc.; and the like. In addition, pivaloyloxymethyl or the like, which is used widely as an ester for oral administration, may also be used.

When the ligand protein of the present invention has a carboxyl group (or a carboxylate) at a position other than the C-terminus, it may be amidated or esterified and such an amide or ester is also included within the ligand protein of the present invention. The ester group may be the same as described with respect to the C-terminus in the above.

Furthermore, the ligand proteins of the present invention include variants of the proteins as described above wherein the amino group at the N-terminal methionine residue is protected with a protecting group (for example, a $C_{1-6}$ acyl group such as a $C_{2-6}$ alkanoyl group, e.g., formyl group, acetyl group, etc.); those wherein the N-terminal region is cleaved *in vivo* to generate glutamyl group, which is then pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{2-6}$ alkanoyl group, e.g., formyl group, acetyl group, etc.), or conjugated proteins such as glycoproteins which has sugar chains.

Examples of the ligand protein of the present invention includes the ligand proteins derived from a mouse, comprising the amino acid sequence represented by SEQ ID NO:1 and NO:2 (mouse type 1 and type 2 ligands, respectively), and the ligand protein derived from a rat, comprising the amino acid sequence represented by SEQ ID NO:3 (rat ligand).

The present invention provides partial peptides of the ligand protein of the present invention (hereinafter sometimes referred to as the partial peptides). These partial peptides may be any one derived from the ligand proteins of the present invention, but should have the property as described above.

Specifically, partial peptides of the protein having the amino acid sequence represented by SEQ ID NO:1, NO:2 or NO:3 includes the mature forms as described above. The partial peptides of these mature partial peptides are also included in the present invention as long as they retain the property as described above.

Examples of partial peptides of the protein having the amino acid sequence represented by SEQ ID NO:1, NO:2 or NO:3 include:

(a) a polypeptide having the amino acid sequence from 90th to 141th residues from the N-terminal of the amino acid sequence represented by SEQ ID NO:1, an amide or ester thereof, or a salt thereof;
(b) a polypeptide comprising the amino acid sequence from 134th to 141th residues from the N-terminal of the amino acid sequence represented by SEQ ID NO:1 and consisting of 8 to 52 amino acids, an amide or ester thereof, or a salt thereof;
(c) a polypeptide having the amino acid sequence from 94th to 145th residues from the N-terminal of the amino acid sequence represented by SEQ ID NO:2, an amide or ester thereof, or a salt thereof;
(d) a polypeptide comprising the amino acid sequence from 138th to 145th residues from the N-terminal of the amino acid sequence represented by SEQ ID NO:2 and consisting of 8 to 52 amino acids, an amide or ester thereof, or a salt thereof;
(e) a polypeptide having the amino acid sequence from 68th to 119th residues from the N-terminal of the amino acid sequence represented by SEQ ID NO:3, an amide or ester thereof, or a salt thereof;
(f) a polypeptide comprising the amino acid sequence from 112th to 119th residues from the N-terminal of the amino acid sequence represented by SEQ ID NO:3 and consisting of 8 to 52 amino acids, an amide or ester thereof, or a salt thereof;
(g) a polypeptide comprising the amino acid sequence from 132th to 141th residues from the N-terminal of the amino acid sequence represented by SEQ ID NO:1, an amide or ester thereof, or a salt thereof;
(h) a polypeptide comprising the same or substantially the same amino acid sequence as the sequence from 127th to 141th residues from the N-terminal of the amino acid sequence represented by SEQ ID NO:1, an amide or ester thereof, or a salt thereof;
(i) a polypeptide comprising the amino acid sequence from 110th to 119th residues from the N-terminal of the amino acid sequence represented by SEQ ID NO:3, an amide or ester thereof, or a salt thereof;
(j) a polypeptide comprising the same or substantially the same amino acid sequence as the sequence from 105th to 119th residues from the N-terminal of the amino acid sequence represented by SEQ ID NO:3, an amide or ester thereof, or a salt thereof.

Among the polypeptides as described in (a) to (j) above, those wherein the C-terminal is an amide form are preferred.

"Substantially the same amino acid sequence" refers to amino acid sequences which have at least about 80% homology, preferably at least about 90% homology, more preferably at least about 95% homology, and most preferably at least about 99% homology to the reference amino acid sequence.

Among those, preferred are the polypeptides, amides or esters thereof, or salts thereof described in (a) to (j) above, and more preferred are:

(i) a polypeptide having the amino acid sequence from 132th to 141th residues from the N-terminal of the amino acid sequence represented by SEQ ID NO:1, an amide or ester thereof, or a salt thereof;
(ii) a polypeptide having the amino acid sequence from 127th to 141th residues from the N-terminal of the amino acid sequence represented by SEQ ID NO:1, an amide or ester thereof, or a salt thereof;
(iii) a polypeptide having the amino acid sequence from 110th to 119th residues from the N-terminal of the amino acid sequence represented by SEQ ID NO:3, an amide or ester thereof, or a salt thereof;
(iv) a polypeptide having the amino acid sequence from 105th to 119th residues from the N-terminal of the amino acid sequence represented by SEQ ID NO:3, an amide or ester thereof, or a salt thereof.

Among these polypeptides, those wherein the C-terminal is an amide form are preferred.

The partial peptide of the present invention may have the amino acid sequences described above, from which at least 1 or 2 (preferably approximately 1 to 10, more preferably several (1 or 2)) amino acids are deleted; to which at least 1 or 2 (preferably approximately 1 to 20, more preferably approximately 1 to 10, and even more preferably several (1 or 2)) amino acids are added; or, in which at least 1 or 2 (preferably approximately 1 to 10, more preferably several (1 or 2)) amino acids are substituted by other amino acids.

In the partial peptide of the present invention, the C-terminus is normally a carboxyl group (-COOH) or carboxylate ($-COO^-$) but the C-terminus may be in the form of an amide ($-CONH_2$) or an ester (-COOR), as described with regard to the ligand protein of the present invention (R has the same definition as above).

As described in the ligand protein of the present invention, the partial peptide of the present invention further includes those in which the amino group of the N-terminal methionine residue is protected by a protecting group, those in which the N-terminal residue is cleaved *in vivo* to generate glutamine residue, which is then pyroglutaminated, those in which substituents on the side chains of amino acids in the molecule are protected by appropriate protecting groups, and also conjugated peptides, such as so-called glycoproteins to which sugar chains are bound, and the like.

The salts of the ligand protein or the partial peptide of the present invention are formed with physiologically acceptable bases or acids. Especially, physiologically acceptable acid addition salts are preferred. Examples of the salts include, for example, salts with inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The ligand protein of the present invention or salts thereof may be produced by a publicly known method for purifying a ligand protein from cells or tissues of a human or other mammals as described above, or by culturing a transformant having the DNA encoding the ligand protein of the present invention, as described below. Furthermore, they may also be produced by a method for synthesizing a protein, as described below, or modified methods thereof.

When the ligand protein or salts thereof are produced from tissues or cells of a human or mammals, these tissues or cells are homogenized, then extracted with an acid or the like, and the extract is subjected to a combination of chromatography techniques, such as reverse phase chromatography, ion exchange chromatography, and the like to isolate and purify the protein.

To synthesize the ligand protein of the present invention, the partial peptide thereof, or salts or amides thereof, commercially available resins for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc. Using these resins, amino acids in which α-amino groups and functional groups on the side chains are appropriately protected are condensed on the resin according to the sequence of the target protein by various condensation methods publicly known in the art. At the end of the reaction, the protein is cut out from the resin and at the same time, the protecting groups are removed. Then, intramolecular disulfide bond-forming reaction is performed in a highly diluted solution to obtain the target protein or its amide.

For condensation of the protected amino acids described above, a variety of activating reagents for protein synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin. Alternatively, the protected amino acids are previously activated in the form of symmetric acid anhydrides, HOBt esters or HOOBt esters, and then thus activated protected amino acids are added to the resin.

Solvents suitable for use to activate the protected amino acids or condense with the resin may be chosen from solvents known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to avoid an adverse effect on the later reaction.

Examples of the protecting groups used to protect the amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting compounds include the corresponding acid anhydrides, azides, activated esters (esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)). As the activated amino acids, in which the amino groups are activated in the starting material, the corresponding phosphoric amides are employed.

To remove(eliminate) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethane-sulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol or 1,4-butanedithiol, as well as by a treatment with an alkali such as a dilute sodium hydroxide solution and dilute ammonia.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining an amide of the protein, for example, the α-carboxyl group of the carboxy terminal amino acid is first protected by amidation; the peptide (protein) chain is then extended from the amino group side to a desired length. Thereafter, a protein in which only the protecting group of the N-terminal α-amino group in the peptide chain has been eliminated from the protein and a protein in which only the protecting group of the C-terminal carboxyl group has been eliminated are prepared. The two proteins are condensed in a mixture of the solvents described above. The details of the condensation reaction are the same as described above. After the protected protein obtained by the condensation is purified, all the protecting groups are eliminated by the method described above to give the desired crude protein. This crude protein is purified by various known purification means. Lyophilization of the major fraction gives the amide of the desired protein.

To prepare the esterified protein, for example, the α-carboxyl group of the carboxy terminal amino acid is condensed with a desired alcohol to prepare the amino acid ester, which is followed by procedure similar to the preparation of the amidated protein above to give the ester form of the desired protein.

The partial peptide of the ligand protein of the present invention can be produced by publicly known methods for peptide synthesis, or by cleaving the protein of the present invention with an appropriate peptidase. For the methods for peptide synthesis, for example, either solid phase synthesis or liquid phase synthesis may be used. That is, the partial peptide or amino acids that can construct the protein of the present invention are condensed with the remaining part. When the product contains protecting groups, these protecting groups are removed to give the desired peptide. Publicly known methods for condensation and elimination of the protecting groups are described in 1) - 5) below.

1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: *Peptide Gosei-no-Kiso to Jikken* (Basics and experiments of peptide synthesis), published by Maruzen Co. (1975)
4) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
5) Haruaki Yajima, ed.: *Zoku Iyakuhin no Kaihatsu* (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide of the present invention. When the partial peptide obtained by the above methods is in a free form, the peptide can be converted into an appropriate salt by a publicly known method; when the protein is obtained in a salt form, it can be converted into a free form by a publicly known method.

The polynucleotide encoding the ligand protein of the present invention may be any polynucleotide so long as it contains the base sequence (DNA or RNA, preferably DNA) encoding the ligand protein of the present invention described above. Such a polynucleotide may also be any one of DNA or RNA such as mRNA encoding the ligand protein of the present invention, and may be double-stranded or single-stranded. When the polynucleotide is double-stranded, it may be double-stranded DNA, double-stranded RNA or DNA:RNA hybrid. When the polynucleotide is single-stranded, it may be a sense strand (i.e. a coding strand) or an antisense strand (i.e. a non-coding strand).

Using the polynucleotide encoding the ligand protein of the present invention, mRNA of the ligand protein of the present invention can be quantified by, for example, the publicly known method published in a separate volume of *Jikken Igaku* 15(7) "New PCR and its application" (1997), or by its modifications.

The DNA encoding the ligand protein of the present invention may be derived from any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

Specifically, the DNA encoding the ligand protein of the present invention may be any DNA having the base sequence shown by SEQ ID NO:4, NO:5 or NO:6, or the base sequence hybridizable to the base sequence represented by SEQ ID NO:4, NO:5 or NO:6 under a high stringent condition, and encoding a ligand protein having the activities substantially equivalent to those of the ligand protein of the present invention (e.g., a ligand binding activity, a signal transduction activity, etc.).

Specific examples of the DNA hybridizable to the base sequence represented by SEQ ID NO:4, NO:5 or NO: 6 include DNA containing a base sequence having at least about 70% homology, preferably at least about 80% homology, more preferably at least about 90% homology and most preferably at least about 95% homology to the base sequence represented by SEQ ID NO:4, NO:5 or NO:6.

The hybridization can be carried out by publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under a high stringent condition.

The high stringent condition refers to, for example, a sodium concentration of about 19 mM to about 40 mM, preferably about 19 mM to about 20 mM and a temperature of about 50°C to about 70°C, preferably about 60°C to about 65°C. In particular, the most preferred condition is a sodium concentration of about 19 mM and a temperature of about 65°C.

More specifically, the DNA encoding the ligand protein having the amino acid sequence represented by SEQ ID NO:1 includes DNA having the base sequence represented by SEQ ID NO:4. The DNA encoding the ligand protein having the amino acid sequence represented by SEQ ID NO:2 includes DNA having the base sequence represented by SEQ ID NO:5. The DNA encoding the ligand protein having the amino acid sequence represented by SEQ ID NO: 3 includes DNA having the base sequence represented by SEQ ID NO:6.

The polynucleotide comprising a part of the base sequence of the DNA encoding the ligand protein of the present invention or a part of the base sequence complementary to the DNA is intended to include not only the DNA encoding the partial peptide of the present invention described below, but also RNA.

According to the present invention, antisense polynucleotides (nucleic acids) that can inhibit the replication or expression of genes of the ligand proteins can be designed and synthesized based on the base sequence information of the cloned or isolated DNAs encoding the proteins. Such a polynucleotide (nucleic acid) is capable of hybridizing to RNA of the gene of the ligand protein to inhibit the synthesis or function of said RNA, or capable of interact with RNA associated with the ligand protein to modulate or control the expression of the gene of the ligand protein. Polynucleotides which is complementary to the selected sequence region of RNA associated with the ligand protein and ones which is specifically hybridizable to RNA associated with the ligand protein are useful in modulating or controlling the expression of gene of the ligand protein *in vivo* and *in vitro,* and thus useful for the treatment or diagnosis of diseases. The term "corresponding" means to be homologous or complementary to a particular nucleotide sequence, base sequence or nucleic acid sequence including a gene. The "corresponding" relation between a nucleotide sequence, base sequence or nucleic acid sequence and a peptide(protein) usually means that amino acids of the peptide (protein) is under control of the nucleotide (nucleic acid) sequence or their complementary sequence. In the gene of ligand protein, the 5'-end hairpin loop, 5'-end 6-base-pair repeats, 5'-end untranslated region, polypeptide translation initiation codon, protein coding region, ORF translation initiation codon, 3'-end untranslated region, 3'-end palindrome region, and 3'-end hairpin loop, may be selected as preferred target regions, though any other region may be selected as a target in the gene of ligand protein.

The relationship between the targeted nucleic acids and the polynucleotides complementary to at least a part of the target, specifically the relationship between the target and the polynucleotides hybridizable to the target, can be denoted to be "antisense". Examples of the antisense polynucleotides include polydeoxynucleotides containing 2-deoxy-D-ribose, polydeoxynucleotides containing D-ribose, any other type of polynucleotides which are N-glycosides of a purine or pyrimidine base, or other polymers containing non-nucleotide backbones (e.g., protein nucleic acids and synthetic sequence-specific nucleic acid polymers commercially available) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., $\alpha$ anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom (s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, increasing the cellular permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain altered or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory activity of the antisense nucleic acid can be examined using the transformant of the present invention, the gene expression system of the present invention *in vivo* and *in vitro,* or the translation system of the ligand protein *in vivo* and *in vitro.* The nucleic acid can be applied to cells by a variety of publicly known methods.

The DNA encoding the partial peptide of the present invention may be any DNA so long as it contains the base sequence encoding the partial peptide of the present invention described above. The DNA may also be derived from any of genomic DNA, genomic DNA library, cDNA derived from the cells and tissues described above, cDNA library derived from the cells and tissues described above and synthetic DNA. The vector to be used for the library may be derived from any of bacteriophage, plasmid, cosmid and phagemid. The DNA may also be directly amplified by reverse transcriptase polymerase chain reaction (hereinafter abbreviated as RT-PCR) using mRNA fraction prepared from the cells and tissues described above.

Specifically, the DNA encoding the partial peptide of the present invention may be any one of, for example, (1) DNA containing a partial base sequence of the DNA having the base sequence represented by SEQ ID NO:4 NO:5 or NO:6, or (2) any DNA containing a partial base sequence of the DNA having a base sequence hybridizable to the base sequence represented by SEQ ID NO:4, NO:5 or NO:6 under a high stringent condition and encoding a ligand protein which has the activities (e.g., a ligand-biding activity, a signal transduction activity, etc.) substantially equivalent to those of the ligand protein of the present invention. Typical examples of DNA containing a partial base sequence of the DNA having the base sequence represented by SEQ ID NO:4, NO:5 or NO:6 include DNAs encoding the mature forms of the ligand proteins of the present invention.

Examples of DNA that is hybridizable to the base sequence represented by SEQ ID NO:4, NO:5 or NO:6 include DNA containing a base sequence having at least about 70% homology, preferably at least about 80% homology, more preferably at least about 90% homology and most preferably at least about 95% homology to the base sequence represented by SEQ ID NO:4, NO:5 or NO:6.

For cloning of the DNA that completely encodes the ligand protein of the present invention or its partial peptide (hereinafter sometimes collectively referred to as the ligand protein of the present invention), the DNA may be either amplified by PCR using synthetic DNA primers containing a part of the base sequence of the ligand protein of the present invention, or the DNA inserted into an appropriate vector can be selected by hybridization with a labeled DNA fragment or synthetic DNA that encodes a part or entire region of the ligand protein of the present invention. The hybridization can be carried out, for example, according to the method described in Molecular Cloning, 2nd, J. Sambrook et al., Cold Spring Harbor Lab. Press, 1989. The hybridization may also be performed using commercially available library in accordance with the protocol described in the attached instructions.

Conversion of the base sequence of the DNA can be effected by the PCR method or a publicly known method such as the Gapped duplex method or the Kunkel method or its modification, using a publicly known kit such as Mutan™-super Express Km (Takara Shuzo Co., Ltd.) or Mutan™-K (Takara Shuzo Co., Ltd.).

The cloned DNA encoding the ligand protein can be used, depending upon purpose, as it is or if desired, for example, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for the ligand protein of the present invention can be produced, for example, by (a) excising the desired DNA fragment from the DNA encoding the ligand protein of the present invention, and then (b) ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ-phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, LTR promoter, CMV promoter, HSV-TK promoter, etc. Among them, CMV promoter or SRα promoter is preferably used.

When the host is bacteria of the genus Escherichia, preferred examples of the promoter include trp promoter, lac promoter, recA promoter, $\lambda P_L$ promoter, lpp promoter, etc. In the case of using bacteria of the genus *Bacillus* as the host, preferred example of the promoter are SPO1 promoter, SPO2 promoter and penP promoter. When yeast is used as the host, preferred examples of the promoter are PHO5 promoter, PGK promoter, GAP promoter and ADH promoter. When insect cells are used as the host, preferred examples of the promoter include polyhedrin prompter and P10 promoter.

In addition to the foregoing examples, the expression vector may further optionally contain an enhancer, a splicing signal, a poly A addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as $Neo^r$, G418 resistance), etc. In particular, when dhfr gene is used as the selection marker in CHO ($dhfr^-$) cells, selection can also be made on thymidine free media.

If necessary and desired, a signal sequence that matches with a host is added to the N-terminus of the ligand protein of the present invention. Examples of the signal sequence that can be used are Pho A signal sequence, OmpA signal sequence, etc. in the case of using bacteria of the genus *Escherichia* as the host; α-amylase signal sequence, subtilisin signal sequence, etc. in the case of using bacteria of the genus *Bacillus* as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing the DNA encoding the receptor protein of the present invention thus constructed, transformants can be produced.

Examples of the host, which may be employed, are bacteria belonging to the genus *Escherichia*, bacteria belonging to the genus *Bacillus*, yeast, insect cells, insects and animal cells, etc.

Specific examples of the bacteria belonging to the genus Escherichia include *Escherichia coli* K12 DH1 (Proc. Natl. Acad. Sci. U.S.A., 60, 160 (1968)), JM103 (Nucleic Acids Research, 9, 309 (1981)), JA221 (Journal of Molecular Biology, 120, 517 (1978)), HB101 (Journal of Molecular Biology, 41, 459 (1969)), C600 (Genetics, 39, 440 (1954)), etc.

Examples of the bacteria belonging to the genus Bacillus include Bacillus subtilis MI114 (Gene, 24, 255 (1983)), 207-21 (Journal of Biochemistry, 95, 87 (1984)), etc.

Examples of yeast include Saccharomyces cereviseae AH22, AH22R$^-$, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vivo, 13, 213-217 (1977).

As the insect, for example, a larva of Bombyx mori can be used (Maeda, et al., Nature, 315, 592 (1985)).

Examples of animal cells include monkey cells COS-7, Vero, Chinese hamster cells CHO (hereinafter referred to as CHO cells), dhfr gene deficient Chinese hamster cells CHO (hereinafter simply referred to as CHO(dhfr$^-$) cell), mouse L cells, mouse AtT-20, mouse myeloma cells, rat GH3, human FL cells, etc.

Bacteria belonging to the genus *Escherichia* can be transformed, for example, by the method described in Proc. Natl. Acad. Sci. U.S.A., 69, 2110 (1972) or Gene, 17, 107 (1982).

Bacteria belonging to the genus *Bacillus* can be transformed, for example, by the method described in Molecular & General Genetics, 168, 111 (1979).

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in *Saibo Kogaku* (Cell Engineering), extra issue 8, *Shin Saibo Kogaku Jikken Protocol* (New Cell Engineering Experimental Protocol), 263-267 (1995), published by Shujunsha, or Virology, 52, 456 (1973).

Thus, the transformant transformed with the expression vector containing the DNA encoding the ligand protein of the present invention can be obtained.

When the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast extracts, vitamins, growth promoting factors etc. may also be added to the medium. Preferably, pH of the medium is adjusted to about 5 to about 8.

A preferred example of the medium for incubation of the bacteria belonging to the genus *Escherichia* is M9 medium supplemented with glucose and Casamino acids (Miller, Journal of Experiments in Molecular Genetics, 431-433, Cold Spring Harbor Laboratory, New York, 1972). If necessary and desired, a chemical such as 3β-indolylacrylic acid can be added to the medium thereby to activate the promoter efficiently.

When the bacteria belonging to the genus Escherichia are used as the host, the transformant is usually cultivated at about 15°C to about 43°C for about 3 hours to about 24 hours. If necessary and desired, the culture may be aerated or agitated.

When the bacteria belonging to the genus Bacillus are used as the host, the transformant is cultivated generally at about 30°C to about 40°C for about 6 hours to about 24 hours. If necessary and desired, the culture can be aerated or agitated.

When yeast is used as the host, the transformant is cultivated, for example, in Burkholder's minimal medium (Bostian, K. L. et al., Proc. Natl. Acad. Sci. U.S.A., 77, 4505 (1980)) or in SD medium supplemented with 0.5% Casamino acids (Bitter, G. A. et al., Proc. Natl. Acad. Sci. U.S.A., 81, 5330 (1984)). Preferably, pH of the medium is adjusted to about 5 to about 8. In general, the transformant is cultivated at about 20°C to about 35°C for about 24 hours to about 72 hours. If necessary and desired, the culture can be aerated or agitated.

When insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary and desired, the culture can be aerated or agitated.

When animal cells are employed as the host, the transformant is cultivated in, for example, MEM medium containing about 5% to about 20% fetal bovine serum (Science, 122, 501 (1952)), DMEM medium (Virology, 8, 396 (1959)), RPMI 1640 medium (The Journal of the American Medical Association, 199, 519 (1967)), 199 medium (Proceeding of the Society for the Biological Medicine, 73, 1 (1950)), etc. Preferably, pH of the medium is adjusted to about 6 to about 8. The transformant is usually cultivated at about 30°C to about 40°C for about 15 hours to about 60 hours and, if necessary and desired, the culture can be aerated or agitated.

As described above, the ligand protein of the present invention or the partial peptide thereof can be produced in the cell membrane of the transformant.

The ligand protein of the present invention or the partial peptide thereof can be separated and purified from the culture described above by the following procedures.

When the ligand protein of the present invention or the partial peptide thereof is extracted from the culture or cells, after cultivation, the transformants or cells are collected by a publicly known method and suspended in a appropriate buffer. The transformants or cells are then disrupted by publicly known methods such as ultrasonication, a treatment with lysozyme and/or freeze-thaw cycling, followed by centrifugation, filtration, etc. Thus, the crude extract of the ligand protein of the present invention or the partial peptide thereof can be obtained. The buffer used for the procedures may contain a protein modifier such as urea or guanidine hydrochloride, or a surfactant such as Triton X-100™, etc. When the ligand protein or the partial peptide thereof is secreted in the culture, after completion of the cultivation, the supernatant can be separated from the transformants or cells,and then can be collected by a publicly known method.

The ligand protein of the present invention or the partial peptide thereof contained in the supernatant or the extract thus obtained can be purified by appropriately combining publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the thus obtained ligand protein or the partial peptide thereof is in a free form, it can be converted into the salt by publicly known methods or modifications thereof. On the other hand, when the receptor protein is obtained in the form of a salt, it can be converted into the free form or in the form of a different salt by publicly known methods or modifications thereof.

The ligand protein or the partial peptide thereof produced by the recombinant can be treated, before or after the purification, with an appropriate protein modifying enzyme so that the protein can be appropriately modified or be deprived of its partial polypeptide. Examples of the protein-modifying enzyme include trypsin, chymotrypsin, arginyl endopeptidase, protein kinase, glycosidase or the like.

The activity of the thus produced ligand protein of the present invention or salts thereof, the partial peptide of the present invention, an ester or amide thereof, or a salt thereof can be determined by a binding assay to a labeled receptor, an enzyme immunoassay using a specific antibody, or the like.

Antibodies to the ligand protein of the present invention or salts thereof, the partial peptide of the present invention, an ester or amide thereof, or a salt thereof (hereinafter sometimes referred to as the ligand protein of the present invention) may be any polyclonal or monoclonal antibodies, as long as they are capable of recognizing the ligand protein of the present invention.

The antibodies to the ligand protein of the present invention may be produced by publicly known methods for producing antibodies or antisera, using as antigens the ligand protein of the present invention.

[Preparation of monoclonal antibody]

(a) Preparation of monoclonal antibody-producing cells

A receptor to the ligand protein of the present invention (hereinafter sometimes referred to as "the receptor") may be administered to mammals either alone or together with carriers or diluents at the site where the production of antibody is possible by the administration. In order to potentiate the antibody productivity upon the administration, complete Freund's adjuvants or incomplete Freund's adjuvants may be administered. The administration is usually carried out once in every two to six weeks and 2 to 10 times in total. Examples of the applicable mammals are monkeys, rabbits, dogs, guinea pigs, mice, rats, sheep and goats, with mice and rats being preferred.

In the preparation of monoclonal antibody-producing cells, warm-blooded animals, e.g., mice, immunized with an antigen wherein the antibody titer is noted is selected, then the spleen or lymph node is collected after 2 to 5 days from the final immunization and antibody-producing cells contained therein are fused with myeloma cells to give monoclonal antibody-producing hybridomas. Measurement of the antibody titer in antisera may be made, for example, by reacting a labeled ligand protein, as described below, with the antiserum, followed by assaying the activity of the labeling agent bound to the antibody. The fusion may be operated, for example, by the known Koehler and Milstein method (Nature, 256, 495, 1975). Examples of the fusion accelerator are polyethylene glycol (PEG), Sendai virus, etc., of which PEG is preferably employed.

Examples of the myeloma cells are NS-1, P3U1, SP2/0, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at about 20 to about 40°C, preferably at about 30 to about 37°C for about 1 to about 10 minutes, an efficient cell fusion can be carried out.

Various methods can be used for screening of a monoclonal antibody-producing hybridoma. Such methods include a method which comprises adding the supernatant of hybridoma to a solid phase (e.g., microplate) adsorbed with the ligand protein of the present invention as an antigen directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase, and a method which comprises adding the supernatant of hybridoma to a solid phase adsorbed with an anti-immunoglobulin antibody or Protein A, adding the ligand protein labeled with a radioactive substance or an enzyme and detecting the monoclonal antibody bound to the solid phase.

The monoclonal antibody can be selected by publicly known methods or by modifications of these methods. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal bovine serum, GIT medium (Wako Pure Chemical Industries, Ltd.) containing 1% to 10% fetal bovine serum, a serum free medium for cultivation of a hybridoma (SFM-101, Nissui Seiyaku Co., Ltd.) and the like can be used for the selection and growth medium. The cultivation is carried out generally at 20°C to 40°C, preferably at about 37°C, for 5 days to 3 weeks, preferably 1 to 2 weeks. The cultivation can be conducted normally in 5% $CO_2$. The antibody titer of the culture supernatant of hybridomas can be determined as in the assay for the antibody titer in antisera described above.

(b) Purification of monoclonal antibody

Separation and purification of a monoclonal antibody can be carried out by methods applied to conventional separation and purification of immunoglobulins, as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

[Preparation of polyclonal antibody]

The polyclonal antibody of the present invention can be produced by publicly known methods or modifications thereof. For example, a complex of an immunogen (the ligand protein as an antigen) and a carrier protein is prepared, and a mammal is immunized with the complex in a manner similar to the method described above for the production of monoclonal antibodies. Materials containing the antibody to the ligand protein or the receptor protein of the present invention is collected from the immunized animal, followed by separation and purification of the antibody.

In the complex of an immunogen and a carrier protein used to immunize a mammal, the type of carrier protein and the mixing ratio of a carrier to hapten may be any type and in any ratio, as long as the antibody is efficiently produced to the hapten immunized by crosslinking to the carrier. For example, bovine serum albumin, bovine thyroglobulins, keyhole limpet hemocyanin, etc. is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to warm-blooded animals either solely or together with carriers or diluents to the site in which the antibody can be produce by the administration. In order to potentiate the antibody productivity upon the administration, complete Freund's adjuvant or incomplete Freund's adjuvant may be administered. The administration is usually made once approximately in every 2 to 6 weeks and about 3 to about 10 times in total.

The polyclonal antibody can be collected from the blood, ascites, etc., preferably from the blood of mammals immunized by the method described above.

The titer of polyclonal antibody in antiserum can be assayed by the same procedure as that for the determination of antibody titer in serum described above. The separation and purification of the polyclonal antibody can be carried out, following the method for the separation and purification of immunoglobulins performed as applied to the separation and purification of monoclonal antibodies described hereinabove.

The ligand protein of the present invention or salts thereof, the partial peptide of the present invention, an ester or amide thereof, or a salt thereof, and the DNA encoding it can be used for: <1> a prophylactic and/or therapeutic agent for diseases associated with dysfunction of the ligand protein of the present invention, <2> an agent for genetic diagnosis, <3> quantification of a receptor to the ligand protein of the present invention, <4> screening for a compound (an agonist, an antagonist, etc.) that alters the binding property between the ligand protein of the present invention and its receptor, <5> a prophylactic and/or therapeutic agent for various diseases comprising a compound (an agonist or an antagonist) that alters the binding property between the ligand protein of the present invention and its receptor, <6> quantification of the ligand protein of the present invention, <7> neutralization by an antibody to the ligand protein of the present invention, and <8> preparation of non-human animals that possess the DNA encoding the ligand protein of the present invention.

In particular, by use of the receptor binding assay, as described below, using the expression system of the recombinant ligand protein of the present invention, a compound (e.g., an agonist, an antagonist) that alters the binding property of the human- or mammal-specific receptor to the ligand protein of the present invention can be screened, and the agonist or antagonist can be used as a prophylactic and therapeutic agent for various diseases.

Use of the ligand protein of the present invention, the DNA encoding it (hereinafter sometimes referred to as the DNA of the present invention) and the antibody to the ligand protein of the present invention (hereinafter sometimes referred to as the antibody of the present invention) is specifically described in the following.

**<1> A prophylactic and/or therapeutic agent for diseases associated with dysfunction of the ligand protein of the present invention**

The ligand protein of the present invention, or the DNA encoding the ligand protein can be used as a prophylactic and/or therapeutic agent for diseases associated with dysfunction of the ligand protein of the present invention.

For example, when a patient cannot rely on the physiological activity of the receptor due to a decrease in the ligand protein of the present invention (deficiency of the ligand protein), the amount of the ligand protein in the patient can be increased and the activity of the receptor to the ligand can be sufficiently induced by (i) administering the ligand protein of the present invention to the patient to supplement the amount of the ligand protein; or (ii) (a) administering the DNA encoding the ligand protein to express the same in the patient; or (b) introducing and expressing the DNA encoding the ligand protein in target cells, and then transplanting the cells to the patient. Thus, the ligand protein of the present invention or the DNA encoding the ligand protein is useful as a safe and low toxic prophylactic and/or therapeutic agent for diseases associated with dysfunction of the ligand protein of the present invention.

The ligand protein of the present invention and the DNA encoding the ligand protein have the activity of inhibiting tumor metastasis, and thus are useful for the prevention and/or treatment of any type of cancers (e.g., cancers of lung, stomach, liver, pancreas, large intestine, rectum, colon, prostate, ovary, uterine cervix, breast, etc.)

Furthermore, an agonist of the receptor protein has the activity of regulating placental function, and thus is useful for the prevention and/or treatment of choriocarcinoma, hydatid mole, invasive mole, miscarriage, fetal dysgenesis, dysbolism of saccharide, dysbolism of lipid, or induction of delivery.

When the ligand protein of the present invention is used as the prophylactic/therapeutic agent, the ligand protein can be prepared into a pharmaceutical composition in a conventional manner.

On the other hand, where the DNA encoding the ligand protein of the present invention (hereinafter sometimes referred to as the DNA of the present invention) is used as the prophylactic/therapeutic agent described above, the DNA itself is administered; alternatively, the DNA is inserted into an appropriate vector such as retrovirus vector, adenovirus vector, adenovirus-associated virus vector, etc. and then administered in a conventional manner. The DNA of the present invention may also be administered as naked DNA, or with adjuvants to assist its uptake by gene gun or through a catheter such as a catheter with a hydrogel.

For example, (i) the ligand protein of the present invention or (ii) the DNA encoding the ligand protein can be used orally, for example, in the form of tablets which may be sugar coated if necessary and desired, capsules, elixirs, microcapsules etc., or parenterally in the form of injectable preparations such as a sterile solution and a suspension in water or with other pharmaceutically acceptable liquid. These preparations can be produced by mixing (i) the ligand protein of the present invention or (ii) the DNA encoding the ligand protein with a physiologically acceptable known carrier, a flavoring agent, an excipient, a vehicle, an antiseptic agent, a stabilizer, a binder, etc. in a unit dosage form required in a generally accepted manner that is applied to making pharmaceutical preparations. The effective component in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated by conventional procedures used to make pharmaceutical compositions, e.g., by dissolving or suspending the active ingredients in a vehicle such as water for injection with a naturally occurring vegetable oil such as sesame oil and coconut oil, etc. to prepare the pharmaceutical composition. Examples of an aqueous medium for injection include physiological saline and an isotonic solution containing glucose and other auxiliary agents (e.g., D-sorbitol, D-mannitol, sodium chloride) and may be used in combination with an appropriate dissolution aid such as an alcohol (e.g., ethanol or the like), a polyalcohol (e.g., propylene glycol and polyethylene glycol), a nonionic surfactant (e.g., polysorbate 80™ and HCO-50), etc. Examples of the oily medium include sesame oil and soybean oil, which may also be used in combination with a dissolution aid such as benzyl benzoate and benzyl alcohol.

The prophylactic/therapeutic agent described above may further be formulated with a buffer (e.g., phosphate buffer, sodium acetate buffer), a soothing agent (e.g., benzalkonium chloride, procaine hydrochloride), a stabilizer (e.g., human serum albumin, polyethylene glycol), a preservative (e.g., benzyl alcohol, phenol), an antioxidant, etc. The thus-prepared liquid for injection is normally filled in an appropriate ampoule.

Since the thus obtained pharmaceutical preparation is safe and low toxic, the preparation can be administered to a human or mammal (e.g., rat, rabbit, sheep, pig, bovine, cat, dog, monkey).

The dose of the ligand protein of the present invention varies depending on subject to be administered, organs to be administered, conditions, routes for administration, etc.; in oral administration, e.g., for a patient with cancer (as 60 kg body weight), the dose is normally about 0.1 mg to about 100 mg, preferably about 1.0 to about 50 mg, and more preferably about 1.0 to about 20 mg per day. In parenteral administration, the single dose varies depending on subject to be administered, target organ, conditions, routes for administration, etc. but it is advantageous, e.g., for a patient with cancer (as 60 kg body weight), to administer the active ingredient intravenously in a daily dose of about 0.01 to about 30 mg, preferably about 0.1 to about 20 mg, and more preferably about 0.1 to about 10 mg. For other animal species, the corresponding dose as converted per 60 kg body weight can be administered.

The dose of the DNA encoding the ligand protein varies depending on subject to be administered, organs to be administered, conditions, routes for administration, etc.; in oral administration, e.g., for a patient with cancer (as 60 kg body weight), the dose is normally about 0.1 mg to about 100 mg, preferably about 1.0 to about 50 mg, and more preferably about 1.0 to about 20 mg per day. In parenteral administration, the single dose varies depending on subject to be administered, target organ, conditions, routes for administration, etc. but it is advantageous, e.g., for a patient with cancer (as 60 kg body weight), to administer the active ingredient intravenously in a daily dose of about 0.01 to about 30 mg, preferably about 0.1 to about 20 mg, and more preferably about 0.1 to about 10 mg. For other animal species, the corresponding dose as converted per 60 kg body weight can be administered.

**<2> An agent for genetic diagnosis**

By using DNA encoding the ligand protein of the present invention or the receptor protein as a probe, an aberration (genetic aberration) of the DNA or mRNA encoding the ligand protein of the present invention in a human or mammal (e.g., rat, rabbit, sheep, pig, bovine, cat, dog, monkey) can be detected. Therefore, the DNA of the present invention is useful as an agent for genetic diagnosis for damage, mutation or decreased expression of the DNA or mRNA, or increased expression or overexpression of the DNA or mRNA.

The genetic diagnosis described above using the DNA encoding the ligand protein of the present invention can be performed by, for example, the publicly known Northern hybridization assay or the PCR-SSCP assay (Genomics, 5, 874-879 (1989); Proceedings of the National Academy of Sciences of the United States of America, 86, 2766-2770 (1989)).

**<3> A method of quantifying the receptor for the ligand protein of the present invention**

Since the receptor protein has binding affinity to the ligand of the present invention, the receptor concentration can be quantified *in vivo* with good sensitivity.

The quantification method of the present invention can be used in combination with, for example, a competitive method. The receptor concentration in a test sample can be measured by contacting the test sample to the ligand protein of the present invention. Specifically, the method can be used by following, for example, the methods described in (i) and (ii) below or modified methods thereof.

(i) Hiroshi Irie, ed. "Radioimmunoassay," Kodansha, published in 1974
(ii) Hiroshi Irie, ed. "Sequel to the Radioimmunoassay," Kodansha, published in 1979.

**<4> A method of screening a compound (agonist, antagonist, or the like) that alters the binding property between the ligand protein of the present invention and the receptor**

Using the receptor protein to the ligand protein of the present invention, or a salt thereof, or using the receptor binding assay system of the expression system constructed using the recombinant receptor protein, a compound (e. g., peptide, protein, non-peptide compound, synthetic compound, fermentation product) or a salt thereof that alters the binding property between the ligand protein of the present invention and the receptor protein can be efficiently screened. Said receptor used herein includes one which is well identified in terms of properties, for example, a G-protein coupled protein, and especially humans mouse or rat OT7T175 is preferred.

Such a compound includes (a) a compound that has the G protein-coupled receptor-mediated cell-stimulating activities (e.g., activities of promoting or suppressing arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction, etc.) (so-called an agonist to the receptor protein); (b) a compound that does not have the cell-stimulating activity (so-called an antagonist to the receptor protein); and (c) a compound that reduces the binding affinity between the ligand protein and the receptor protein.

Thus, the present invention provides a method of screening a compound or a salt thereof that alters the binding property between the ligand protein of the present invention and the receptor protein, which comprises comparing (i) the case wherein the ligand protein of the present invention is brought in contact with the receptor protein, with (ii) the case wherein the ligand protein is brought in contact with the receptor and a test compound.

The screening method of the present invention is characterized by assaying, for example, the binding amount of the ligand to the receptor, the cell-stimulating activity, and comparing the assay results of (i) and (ii).

More specifically, the present invention provides the following screening methods:

(1) a method of screening a compound or a salt thereof that alters the binding property between the ligand protein of the present invention and the receptor protein, which comprises:

measuring the binding amount of the labeled ligand to the receptor protein, when the labeled ligand is brought in contact with the receptor protein and when the labeled ligand and a test compound are brought in contact with the receptor protein, and,
comparing their binding amounts, (in this case, the labeled receptor can replace the labeled ligand);

(2) a method of screening a compound or a salt that alters the binding property between the ligand protein and the receptor protein, which comprises:

measuring the binding amount of the labeled ligand to a cell containing the receptor protein or the membrane fraction thereof, when the labeled ligand is brought in contact with the cell or cell membrane fraction and when the labeled ligand and a test compound are brought in contact with the cell or cell membrane fraction, and
comparing their binding amounts;

(3) a method of screening a compound or a salt thereof that alters the binding property between the ligand protein and the receptor protein, which comprises:

measuring the binding amount of the labeled ligand to the receptor protein expressed on a cell membrane of a transformant culture containing the DNA of the present invention, when the labeled ligand is brought in contact with the receptor protein expressed and when the labeled ligand and a test compound are brought in contact with the receptor protein expressed, and,
comparing their binding amounts;

(4) a method of screening a compound or a salt thereof that alters the binding property between the ligand protein and the receptor protein, which comprises:

measuring the receptor-mediated cell-stimulating activity (e.g., activity of promoting or suppressing arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction, etc.), when the labeled ligand is brought in contact with a cell containing the receptor protein and when the labeled ligand and a test compound are brought in contact with the cell, and,
comparing their activities; and

(5) a method of screening a compound or a salt thereof that alters the binding property between the ligand protein and the receptor protein, which comprises:

measuring the receptor-mediated cell-stimulating activity (e.g., activity of promoting or suppressing arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction, etc.), when the labeled ligand is brought in contact with the receptor protein expressed on a cell membrane of a transformant culture containing the DNA of the present invention and when the labeled ligand and a test compound are brought in contact with the receptor protein expressed, and,
comparing their activities.

Using any one of the methods (1) to (5), a compound that inhibits the binding of the ligand and the receptor can be efficiently screened. Furthermore, it is easy to assess whether the obtained compound is an agonist or antagonist.

More specifically, the screening methods of the present invention are described hereinafter.

First, for the receptor protein used for the screening methods of the present invention, any substance may be used so long as it contains the receptor protein as described above. The cell membrane fraction from mammalian organs containing the receptor protein is preferred. However, it is preferable to use the receptor protein produced in a large amount using a recombinant for the screening.

To produce the receptor protein, the methods described above are used, and it is preferred to express a DNA encoding the receptor protein in mammalian and insect cells. For the DNA fragment encoding the target protein, the complementary DNA, but not necessarily limited thereto, is employed. For example, the gene fragments and synthetic DNA may also be used. To introduce a DNA fragment encoding the receptor protein into host animal cells and efficiently express the DNA there, it is preferred to insert the DNA fragment downstream of a polyhedorin promoter of nuclear polyhedrosis virus (NPV) belonging to baculovirus hosted by insects, SV40-derived promoter, retrovirus promoter, metallothionein promoter, human heat shock promoter, cytomegalovirus promoter, or SRα promoter. The amount and quality of the expressed receptor are examined by publicly known methods, for example, the method described in the literature [Nambi, P. et al., The Journal of Biological Chemistry (J. Biol. Chem.), 267, 19555-19559, 1992].

Therefore, in the screening methods of the present invention, the material containing the receptor protein may be the receptor protein purified by publicly known methods, a cell containing the receptor protein, or the cell membrane fraction containing the receptor protein.

When the cell containing the receptor protein is used for the screening method of the present invention, the cell may be fixed with glutaraldehyde, formalin, etc. The fixation can be made by publicly known methods.

The cell containing the receptor protein refers to a host cell that expresses the receptor protein. For the host cell, *Escherlchia coli*, *Bacillus subtilis*, yeast, insect cells, animal cells are preferred.

The cell membrane fraction refers to a fraction abundant in cell membrane obtained by cell disruption and subsequent fractionation by a publicly known method. Useful cell disruption methods include cell squashing using a Potter-Elvehjem homogenizer, disruption using a Waring blender or Polytron (produced by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein expressed and membrane components such as cell-derived phospholipids and membrane proteins.

The amount of the receptor protein in the cell or the membrane fraction containing the receptor protein is preferably $10^3$ to $10^8$ molecules per cell, more preferably $10^5$ to $10^7$ molecules per cell. As the amount of expression increases, the ligand binding activity per unit of membrane fraction (specific activity) increases so that not only the highly sensitive screening system can be constructed but also large quantity of samples can be assayed with the same lot.

To perform the screening methods (1) to (3) for a compound that alters the binding property between the ligand protein of the present invention and the receptor protein, for example, an appropriate receptor protein fraction and the labeled ligand are necessary.

The receptor protein fraction is preferably a fraction of naturally occurring receptor protein or a recombinant receptor fraction having an activity equivalent to that of the natural protein. Herein, the equivalent activity means a ligand binding activity, a signal transduction activity or the like.

The labeled ligand includes a labeled ligand and a labeled ligand analogue. For example, the ligand labeled with [$^3$H], [$^{125}$I], [$^{14}$C], [$^{35}$S], etc. are used (the labeled ligand protein of the present invention).

Specifically, for the screening for a compound that alters the binding property between the ligand protein and the receptor protein, first, the receptor protein standard is prepared by suspending the cell or cell membrane fraction containing the receptor protein in a buffer appropriate for the screening. For the buffer, any buffer that does not interfere with the binding of the ligand to the receptor is usable and examples of such a buffer are phosphate buffer, Tris-hydrochloride buffer, etc., having pH of 4 to 10 (preferably pH of 6 to 8). To minimize a non-specific binding, a surfactant such as CHAPS, Tween-80™ (Kao-Atlas Co.), digitonin, deoxycholate, etc. may be added to the buffer. To inhibit degradation of the receptor and the ligand protein by proteases, protease inhibitors such as PMSF, leupeptin, E-64 (produced by Peptide Research Laboratory, Co.), and pepstatin may be added. To 0.01 to 10 ml of the receptor solution, a given amount (5,000 to 500,000 cpm) of the labeled ligand is added, and $10^{-4}$ M - $10^{-10}$ M of a test compound is simultaneously added thereto. To examine non-specific binding (NSB), a reaction tube containing excess of the unlabeled ligand is also prepared. The reaction is carried out at approximately 0 to 50°C, preferably about 4 to 37°C for about 20 minutes to about 24 hours, preferably about 30 minutes to about 3 hours. After completion of the reaction, the reaction mixture is filtrated through glass fiber filter paper, etc. and washed with an appropriate volume of the same buffer. The residual radioactivity on the glass fiber filter paper is then measured by means of a liquid scintillation counter or γ-counter. Regarding the count obtained by subtracting the amount of non-specific binding (NSB) from the count obtained in the absence of any competitive substance ($B_0$) as 100%, when the amount of specific binding (B-NSB) is, for example, 50% or less, the test compound can be selected as a candidate substance having a potential of competitive inhibition.

To perform the screening methods (4) and (5) supra for a compound that alters the binding property between the ligand protein and the receptor protein, the receptor protein-mediated cell-stimulating activity (e.g., activity of promoting or inhibiting arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction, etc.) can be measured using publicly known methods or commercially available kits.

Specifically, the cell containing the receptor protein is first cultured on a multi-well plate, etc. Prior to screening, the medium is replaced with fresh medium or with an appropriate non-cytotoxic buffer, followed by incubation for a given period of time in the presence of a test compound. Subsequently, in the extract from the cells or the supernatant recovered, any resulting product is quantified by appropriate procedures. When it is difficult to detect the production of the index substance (e.g., arachidonic acid) for the cell-stimulating activity, due to a degrading enzyme contained in the cells, an inhibitor against such a degrading enzyme may be added prior to the assay. For detecting activities such as the cAMP production suppression activity, the baseline production in the cell is increased by forskolin or the like and the suppressing effect on the increased baseline production may then be detected.

The screening by assaying the cell-stimulating activity requires a cell expressing an appropriate receptor protein. For the cell expressing the receptor protein, a cell line possessing the native receptor protein, a cell line expressing the recombinant receptor protein described above and the like are desirable.

For a test compound, for example, a peptide, protein, non-peptide compound, synthetic compound, fermentation product, cell extract, plant extract, and animal tissue extract are used. The compound may be novel or known.

The kit for screening a compound or a salt thereof that alters the binding property between the ligand protein and the receptor protein comprises the receptor protein, a cell containing the receptor protein, or a membrane fraction of the cell containing the receptor protein.

Examples of the screening kits of the present invention are as follow.

1. Reagents for the screening

(i) Buffer for measurement and washing
Hanks' balanced salt solution (Gibco Co.) supplemented with 0.05% bovine serum albumin (Sigma Co.).
The solution is sterilized by filtration through a 0.45 μm filter, and stored at 4°C or may be prepared at use.
(ii) Standard G protein-coupled receptor CHO cells expressing the receptor protein are passaged in a 12-well plate at a density of $5 \times 10^5$ cells/well followed by culturing at 37°C under 5% $CO_2$ and 95% air for 2 days.
(iii) Labeled ligand
An aqueous solution of the ligand labeled with commercially available [$^3$H], [$^{125}$I], [$^{14}$C], [$^{35}$S], etc. is stored at 4°C or -20°C, and diluted to 1 μM with the measurement buffer at use.
(iv) Standard ligand solution
The ligand protein of the present invention is dissolved in and adjusted to 1 mM with PBS containing 0.1% bovine serum albumin (Sigma Co.) and stored at -20°C.

2. Measurement method

(i) CHO cells expressing the receptor protein are cultured in a 12-well culture plate and washed twice with 1 ml of the measurement buffer, and 490 μl of the measurement buffer is added to each well.

(ii) After adding 5 μl of $10^{-3}$ - $10^{-10}$ M test compound solution, 5 μl of the labeled ligand is added to the mixture, and the cells are incubated at room temperature for an hour. To determine the amount of the non-specific binding, 5 μl of the non-labeled ligand is added in place of the test compound.

(iii) The reaction solution is removed, and the wells are washed 3 times with the washing buffer. The labeled ligand bound to the cells is dissolved in 0.2N NaOH-1% SDS, and mixed with 4 ml of liquid scintillator A(Wako Pure Chemical Industries, Ltd.)

(iv) The radioactivity is measured using a liquid scintillation counter(Beckman Co.), and the percent maximum binding (PMB) is calculated by the equation below.

$$PMB = [(B - NSB)/(B_0 - NSB)] \times 100$$

PMB : Percent maximum binding
B : Value obtained in the presence of a test compound
NSB : Non-specific binding
$B_0$ : Maximum binding

The compound or its salt, which is obtainable using the screening method or the screening kit of the present invention, is one that alters the binding property between the ligand protein of the present invention and the receptor protein. Specifically, the compound is: (a) a compound having the G protein-coupled receptor-mediated cell-stimulating activity (e.g., activity of promoting or inhibiting arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, pH reduction) (so-called an agonist to the receptor protein); (b) a compound having no cell stimulating-activity (so-called an antagonist to the receptor protein); or (c) a compound that reduces the binding affinity between the ligand protein of the present invention and the receptor protein.

The compound may be a peptide, protein, non-peptide compound, synthetic compound, fermentation product, and may be novel or known.

Since an agonist to the receptor protein has the same physiological activity as that of the ligand of the present invention to the receptor protein, it is useful as a safe and lo-toxic pharmaceutical, depending on the ligand activity.

Specifically, an agonist of the receptor protein has the activity of inhibiting tumor metastasis, and thus is useful for the prevention and/or treatment of any type of cancers (e.g., cancers of lung, stomach, liver, pancreas, large intestine, rectum, colon, prostate, ovary, uterine cervix, breast, etc.)

Furthermore, an agonist of the receptor protein has the activity of regulating placental function, and thus is useful for the prevention and/or treatment of choriocarcinoma, hydatid mole, invasive mole, miscarriage, fetal dysgenesis, dysbolism of saccharide, dysbolism of lipid, or induction of delivery.

Since an antagonist to the receptor protein can suppress the physiological activities of the ligand protein to the receptor protein, it is useful as a safe and low-toxic pharmaceutical to inhibit the ligand activity.

The compound that reduces the binding affinity between the ligand protein of the present invention and the receptor protein is useful as a safe and low-toxic pharmaceutical to decrease the physiological activity of the ligand protein to the receptor receptor.

When a compound or its salt, which is obtainable using the screening method or the screening kit of the present invention, is employed as a pharmaceutical composition, the composition can be prepared in a conventional manner. For example, the compound can be formulated into a tablet, capsule, elixir, microcapsule, aseptic solution, suspension, etc., as described for the pharmaceutical containing the DNA of the present invention.

The preparation thus obtained is safe and low-toxic, and can be administered to, for example, a human and mammal (e.g., rat, rabbit, sheep, pig, bovine, cat, dog, monkey).

The dose of the compound or its salt varies depending on subject to be administered, target organs, conditions, routes for administration, etc. In oral administration, e.g., to a patient with cancer (as 60 kg body weight), the dose is normally about 0.1 mg to about 100 mg, preferably about 1.0 to about 50 mg, and more preferably about 1.0 to about 20 mg per day. In parenteral administration, the single dose varies depending on a subject to be administered, target organ, conditions, routes for administration, etc. but it is advantageous, e.g., for a patient with cancer (as 60 kg body weight), to administer the active ingredient intravenously in a daily dose of about 0.01 to about 30 mg, preferably about 0.1 to about 20 mg, and more preferably about 0.1 to about 10 mg. For other animal species, the corresponding dose as converted per 60 kg body weight can be administered.

**<5> A prophylactic and/or therapeutic agent for various diseases comprising a compound (agonist or antagonist) that alters the binding property between the ligand protein of the present invention and the receptor**

As described above, the ligand protein of the present invention has the activity of inhibiting tumor metastasis, and thus is useful for the prevention and/or treatment of any type of cancers (e.g., cancers of lung, stomach, liver, pancreas, large intestine, rectum, colon, prostate, ovary, uterine cervix, breast, etc.)

Furthermore, an agonist of the receptor protein has the activity of regulating placental function, and thus is useful for the prevention and/or treatment of choriocarcinoma, hydatid mole, invasive mole, miscarriage, fetal dysgenesis, dysbolism of saccharide, dysbolism of lipid, or induction of delivery.

Therefore, the compound (agonist or antagonist) that alters the binding property between the ligand protein of the present invention and the receptor can be used as a prophylactic and/or therapeutic agent for diseases associated with dysfunction, deficiency or excess of the ligand protein of the present invention.

When the compound is used as the prophylactic and/or therapeutic agent for diseases associated with dysfunction, deficiency or excess of the ligand protein of the present invention, the formulation can be made in a conventional manner.

For example, the compound can be administered orally as sugar coated tablet, capsule, elixir, and microcapsule, or non-orally as injection such as aseptic solution or suspension in water or other pharmaceutically acceptable liquid. For example, these formulations can be produced by mixing the compound with a physiologically acceptable known carrier, flavor, filler, vehicle, antiseptic, stabilizer, and binder in a unit-dosage form generally approved for drug preparation. The amount of the effective ingredient is set to an appropriate amount within the specified range.

An additive that may be mixed in a tablet, capsule, etc. includes, for example, binders such as gelatin, cornstarch, tragacanth, and acacia, fillers such as crystalline cellulose, imbibers such as cornstarch, gelatin, and alginic acid, lubricants such as magnesium stearate, sweeteners such as sucrose and saccharin, and flavors such as peppermint, akamono oil and cherry. When the dosage form is a capsule, liquid carrier such as fat and oil may be contained. An aseptic composition for injection can be prepared following the conventional technique for the preparation, such as by dissolving or suspending the active substance in a vehicle, e.g., water for injection, and natural plant oil e.g., sesame oil and coconut oil. For the aqueous solution for injection, for example, a physiological saline and isotonic solution (e.g., D-sorbitol, D-mannitol, sodium hydrochloride) containing glucose and other adjuvant are used. An appropriate dissolution-assisting agent, for example, alcohol (e.g., ethanol), polyalcohol (e.g., propylene glycol, polyethylene glycol), nonionic surfactant (e.g., polysorbate 80™, HCO-50) may be combined. For the oily solution, for example, sesame oil and soybean oil are used, and dissolution-assisting agents such as benzyl benzoate and benzyl alcohol may be combined.

The prophylactic/therapeutic agent as described above may be combined, for example, with a buffer (e.g., phosphate buffer, sodium acetate buffer), soothing agent (e.g., benzalkonium chloride, procaine hydrochloride), stabilizer (e.g., human serum albumin, polyethylene glycol), preservative (e.g., benzyl alcohol, phenol), and antioxidant. The prepared injection solution is usually filled in an appropriate ampoule.

The preparation thus obtained is safe and low toxic, and can be administered to, for example, a human and mammal (e.g., rat, rabbit, sheep, pig, bovine, cat, dog, monkey).

The dose of the compound or its salt varies depending on the subject to be administered, target organs, conditions, routes for administration, etc. In oral administration, e.g., to a patient with cancer (as 60 kg body weight), the dose is normally about 0.1 mg to about 100 mg, preferably about 1.0 to about 50 mg, and more preferably about 1.0 to about 20 mg per day. In parenteral administration, the single dose varies depending on the subject to be administered, target organ, conditions, routes for administration, etc. but it is advantageous, e.g., for a patient with cancer (as 60 kg body weight), to administer the active ingredient intravenously in a daily dose of about 0.01 to about 30 mg, preferably about 0.1 to about 20 mg, and more preferably about 0.1 to about 10 mg. For other animal species, the corresponding dose as converted per 60 kg body weight can be administered.

**<6> Quantification of the ligand protein of the present invention**

The antibody to ligand protein or receptor protein of the present invention is capable of specifically recognizing the ligand protein or receptor protein of the present invention. Therefore, the antibody can be used to quantify the ligand protein or receptor protein of the present invention in a test fluid, especially for quantification by the sandwich immunoassay. Thus, the present invention provides, for example, the following quantification methods:

(i) a method of quantifying the ligand protein or receptor protein of the present invention in a test fluid, which comprises competitively reacting the antibody to the ligand protein or receptor protein with the test fluid and a labeled form of the ligand protein or receptor protein, and measuring the ratio of the labeled ligand protein or receptor protein bound to the antibody; and

(ii) a method of quantifying the ligand protein or receptor protein of the present invention in a test fluid, which comprises reacting the test fluid with the antibody to the ligand protein or receptor protein immobilized on a carrier and a labeled form of the antibody simultaneously or sequentially, and measuring the activity of the label on the immobilized carrier.

In (ii) described above, it is preferred that one antibody recognizes the N-terminal region of the ligand protein or receptor protein of the present invention, and another antibody reacts with the C-terminal region of the ligand protein or receptor protein.

Using a monoclonal antibody to the ligand protein or receptor protein of the present invention (hereinafter sometimes referred to as the monoclonal antibody of the present invention), the ligand protein or receptor protein of the present invention can be assayed and also detected by tissue staining or the like. For this purpose, the antibody molecule itself may be used, or $F(ab')_2$, Fab' or Fab fractions of the antibody molecule may also be used. The type of assay method using the antibody to the ligand protein or receptor protein of the present invention is not particularly limited. Any assay method can be used, so long as the amount of antibody, antigen, or antibody-antigen complex, corresponding to the amount of antigen (e.g., the amount of the receptor protein) in the test fluid, can be detected by chemical or physical means, and then the amount of the antigen can be calculated from a standard curve prepared from standard solutions containing known amounts of the antigen. For example, nephrometry, competitive methods, immunometric method, and sandwich method are appropriately used, with the sandwich method described below being most preferable in terms of sensitivity and specificity.

As the labeling agent for the methods using labeled substances, there are employed, for example, radioisotopes, enzymes, fluorescent substances, luminescent substances, etc. For the radioisotope, for example, [$^{125}I$], [$^{131}I$], [$^{3}H$] and [$^{14}C$] are used. As the enzyme described above, stable enzymes with high specific activity are preferred; for example, β-galactosidase, β-glucosidase, alkaline phosphatase, peroxidase, malate dehydrogenase and the like are used. Example of the fluorescent substance used are fluorescamine and fluorescein isothiocyanate are used. For the luminescent substance, for example, luminol, luminol derivatives, luciferin, and lucigenin. Furthermore, the biotin-avidin system may be used for binding antibody or antigen to the label.

For immobilization of antigen or antibody, physical adsorption may be used. Chemical binding methods conventionally used for insolubilization or immobilization of proteins or enzymes may also be used. For the carrier, for example, insoluble polysaccharides such as agarose, dextran, cellulose, etc.; synthetic resin such as polystyrene, polyacrylamide, silicon, etc., and glass or the like. are used.

In the sandwich method, the immobilized monoclonal antibody of the present invention is reacted with a test fluid (primary reaction), then with the labeled monoclonal antibody of the present invention (secondary reaction), and the activity of the label on the immobilizing carrier is measured, whereby the amount of the ligand protein or receptor protein of the present invention in the test fluid can be quantified. The order of the primary and secondary reactions may be reversed, and the reactions may be performed simultaneously or with an interval. The methods of labeling and immobilization can be performed by the methods described above.

In the immunoassay by the sandwich method, the antibody used for immobilized or labeled antibodies is not necessarily one species, but a mixture of two or more species of antibody may be used to increase the measurement sensitivity.

In the methods of assaying the ligand protein or receptor protein of the present invention by the sandwich method, antibodies that bind to different sites of the ligand protein are preferably used as the monoclonal antibodies of the present invention for the primary and secondary reactions. That is, in the antibodies used for the primary and secondary reactions are, for example, when the antibody used in the secondary reaction recognizes the C-terminal region of the receptor protein, it is preferable to use the antibody recognizing the region other than the C-terminal region for the primary reaction, e.g., the antibody recognizing the N-terminal region.

The monoclonal antibody to the ligand protein or receptor protein of the present invention can be used for the assay systems other than the sandwich method, for example, competitive method, immunometric method, nephrometry, etc. In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying these immunological methods to the measurement methods of the present invention, any particular conditions or procedures are not required. Systems for measuring the ligand protein or receptor protein of the present invention are constructed by adding the usual technical consideration in the art to the conventional conditions and procedures. For the details of these general technical means, reference can be made to the following reviews and texts. [For example, Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immonoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies)) (all published by Academic Press Publishing).

As described above, the ligand protein or receptor protein of the present invention can be quantified with high sensitivity, using the antibody of the present invention.

By quantifying the ligand protein or receptor protein of the present invention *in vivo* using the antibody of the present invention, diagnosis can be made on various diseases associated with dysfunction of the ligand protein of the present invention.

The antibody to the ligand protein or receptor protein of the present invention can also be used for specifically detecting the ligand protein or receptor protein of the present invention present in test samples such as body fluids or tissues. The antibody may also be used for preparation of antibody columns for purification of the ligand protein or receptor protein of the present invention, for detection of the ligand protein of the present invention in each fraction upon purification, and for analysis of the behavior of the ligand protein or receptor protein of the present invention in the test cells.

**<7> Neutralization with the antibody to the ligand protein of the present invention**

The neutralizing activity of the antibody to the ligand protein or receptor protein of the present invention refers to an activity of inactivating the signal transduction function involving the ligand protein or receptor protein. Therefore, when the antibody has the neutralizing activity, the antibody can inactivate the signal transduction involving the ligand protein or receptor protein, for example, inactivate the ligand protein-mediated cell-stimulating activity (e.g., activity of promoting or inhibiting arachidonic acid release, acetylcholine release, intracellular $Ca^{2+}$ release, intracellular cAMP production, intracellular cGMP production, inositol phosphate production, changes in cell membrane potential, phosphorylation of intracellular proteins, activation of c-fos, decrease in pH, etc.). Therefore, the antibody can be used for the prevention and/or treatment of diseases caused by overexpression of the ligand protein or receptor protein.

**<8> Preparation of animals carrying the DNA encoding the ligand protein of the present invention**

Using the DNA of the present invention, transgenic animals expressing the ligand protein of the present invention can be prepared. Examples of the animals include mammals (e.g., rats, mice, rabbits, sheep, swine, bovine, cats, dogs, monkeys, etc.) can be used, with mice and rabbits being particularly appropriate.

To transfer the DNA of the present invention to target animals, it is generally advantageous to use a gene construct in which the DNA is ligated downstream of a promoter suitable for expression in animal cells. For example, when the DNA of the present invention derived from a mouse is transferred, a gene construct in which the DNA is ligated downstream of a promoter, which can express DNAs of animals highly homologous to the DNA, is microinjected to, for example, a mouse fertilized egg; thus, the DNA-transferred animal, which is capable of producing a high level of the receptor protein of the present invention, can be produced. Examples of the promoters that are usable include virus-derived promoters and ubiquitous expression promoters such as metallothionein promoter, but promoters of NGF gene and enolase that are specifically expressed in the brain are preferably used.

The transfer of the DNA of the present invention at the fertilized egg cell stage secures the presence of the DNA in all germ and somatic cells in the produced animal. The presence of the ligand protein of the present invention in the germ cells in the DNA-transferred animal means that all germ and somatic cells contain the ligand protein of the present invention in all progenies of the animal. The progenies of the animal that took over the gene contain the ligand protein of the present invention in all germ and somatic cells.

The DNA-transferred animals of the present invention can be maintained and bled in the conventional environment as animals carrying the DNA after confirming the stable retention of the gene in the animals through mating. Furthermore, mating male and female animals containing the objective DNA results in acquiring homozygote animals having the transferred gene on both homologous chromosomes. By mating the male and female homozygotes, bleeding can be performed so that all progenies contain the DNA.

Since the ligand protein of the present invention is highly expressed in the animals in which the DNA of the present invention has been transferred, the animals are useful for screening of agonists or antagonists to the ligand protein of the present invention.

The animals in which the DNA of the present invention has been transferred can also be used as cell sources for tissue culture. The ligand protein of the present invention can be analyzed by, for example, directly analyzing the DNA or RNA in tissues from the mouse in which the DNA of the present invention has been transferred, or by analyzing tissues containing the ligand protein expressed from the gene. Cells from tissues containing the ligand protein of the present invention are cultured by the standard tissue culture technique. Using these cells, for example, the function of tissue cells such as cells derived from the brain or peripheral tissues, which are generally difficult to culture, can be studied. Using these cells, for example, it is possible to select pharmaceuticals that increase various tissue functions. When a highly expressing cell line is available, the ligand protein of the present invention can be isolated and purified from the cell line.

In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA : | deoxyribonucleic acid |
| cDNA : | complementary deoxyribonucleic acid |
| A: | adenine |
| T : | thymine |
| G : | guanine |
| C: | cytosine |
| RNA : | ribonucleic acid |
| mRNA : | messenger ribonucleic acid |
| dATP : | deoxyadenosine triphosphate |
| dTTP : | deoxythymidine triphosphate |
| dGTP : | deoxyguanosine triphosphate |
| dCTP : | deoxycytidine triphosphate |
| ATP : | adenosine triphosphate |
| EDTA : | ethylenediaminetetraacetic acid |
| SDS : | sodium dodecyl sulfate |
| Gly : | glycine |
| Ala : | alanine |
| Val : | valine |
| Leu : | leucine |
| Ile : | isoleucine |
| Ser : | serine |
| Thr : | threonine |
| Cys : | cysteine |
| Met : | methionine |
| Glu : | glutamic acid |
| Asp : | aspartic acid |
| Lys : | lysine |
| Arg : | arginine |
| His : | histidine |
| Phe : | phenylalanine |
| Tyr : | tyrosine |
| Trp : | tryptophan |
| Pro : | proline |
| Asn : | asparagine |
| Gln : | glutamine |
| pGlu : | pyroglutamic acid |
| Me: | methyl |

| | |
|---|---|
| Et : | ethyl |
| Bu: | butyl |
| Ph: | phenyl |
| TC: | thiazolidine-4(R)-carboxamide |

The substituents, protective groups and reagents, which are frequently used throughout the specification, are shown by the following abbreviations.

| | |
|---|---|
| Tos : | p-toluenesulfonyl |
| CHO : | formyl |
| Bzl : | benzyl |
| $Cl_2Bl$: | 2,6-dichlorobenzyl |
| Bom : | benzyloxymethyl |
| Z: | benzyloxycarbonyl |
| Cl-Z : | 2-chlorobenzyloxycarbonyl |
| Br-Z : | 2-bromobenzyloxycarbonyl |
| Boc : | t-butoxycarbonyl |
| DNP : | dinitrophenol |
| Trt : | trityl |
| Bum : | t-butoxymethyl |
| Fmoc : | N-9-fluorenylmethoxycarbonyl |
| HOBt : | 1-hydroxybenztriazole |
| HOOBt: | 3,4-dihydro-3-hydroxy-4-oxo-1,2,3-benzotriazine |
| HONB : | 1-hydroxy-5-norbornene-2,3-dicarboximide |
| DCC : | N,N'-dicyclohexylcarbodiimide |
| BHA : | benzhydrylamine |
| MeBzl: | 4-methylbenzyl |
| OcHex: | cyclohexylester |
| NMP : | N-methylpyrrolidone |
| TFA : | trifluoroacetic acid |

The sequence identification numbers (SEQ ID NO) in the sequence listing of the specification indicates the following sequence, respectively.

[SEQ ID NO:1]

This shows the amino acid sequence of a novel mouse ligand protein KiSS-1 of the present invention (mouse type 1).

[SEQ ID NO:2]

This shows the amino acid sequence of a novel mouse ligand protein KiSS-1 of the present invention (mouse type 2).

[SEQ ID NO:3]

This shows the amino acid sequence of a novel rat ligand protein KiSS-1 of the present invention (rat type).

[SEQ ID NO:4]

This shows the cDNA sequence encoding a novel mouse ligand protein KiSS-1 of the present invention (mouse type 1).

[SEQ ID NO:5]

This shows the cDNA sequence encoding a novel mouse ligand protein KiSS-1 of the present invention (mouse type 2).

[SEQ ID NO:6]

This shows the cDNA sequence encoding a novel rat ligand protein KiSS-1 of the present invention (rat type).

[SEQ ID NO:7]

This shows the amino acid sequence of a novel human G protein-coupled receptor protein hOT7T175.

[SEQ ID NO:8]

This shows the amino acid sequence of a novel rat G protein-coupled receptor protein rOT7T175.

[SEQ ID NO:9]

This shows the nucleic acid sequence of primer A used for cloning a mouse KiSS-1 of the present invention.

[SEQ ID NO:10]

This shows the nucleic acid sequence of primer B used for cloning a mouse KiSS-1 of the present invention.

[SEQ ID NO:11]

This shows the nucleic acid sequence of primer C used for cloning a mouse KiSS-1 of the present invention.

[SEQ ID NO:12]

This shows the nucleic acid sequence of primer D used for cloning a mouse KiSS-1 of the present invention.

[SEQ ID NO:13]

This shows the nucleic acid sequence of primer E used for cloning a mouse KiSS-1 of the present invention.

[SEQ ID NO:14]

This shows the nucleic acid sequence of a degenerate primer 13-3F38 used for cloning cDNA encoding the rat ligand (1-54) [rat KiSS-1] of the present invention.

[SEQ ID NO:15]

This shows the nucleic acid sequence of a degenerate primer KiSS357R used for cloning cDNA encoding the rat ligand (1-54) [rat KiSS-1] of the present invention.

[SEQ ID NO:16]

This shows the nucleic acid sequence of DNA fragment encoding a partial peptide of rat KiSS-1.

[SEQ ID NO:17]

This shows the nucleic acid sequence of primer 288-41F for obtaining DNA fragment encoding the entire peptide of rat KiSS-1.

[SEQ ID NO:18]

This shows the nucleic acid sequence of primer 288-10F for obtaining DNA fragment encoding the entire peptide of rat KiSS-1.

[SEQ ID NO:19]

This shows the nucleic acid sequence of primer 288-254R for obtaining DNA fragment encoding the entire peptide of rat KiSS-1.

[SEQ ID NO:20]

This shows the nucleic acid sequence of primer 288-44R for obtaining DNA fragment encoding the entire peptide of rat KiSS-1.

[SEQ ID NO:21]

This shows the nucleic acid sequence of primer rKiSS364F for obtaining DNA fragment encoding the entire peptide of rat KiSS-1.

[SEQ ID NO:22]

This shows the nucleic acid sequence of primer rKiSS859R for obtaining DNA fragment encoding the entire peptide of rat KiSS-1.

[SEQ ID NO:23]

This shows the nucleic acid sequence of 393bp DNA fragment encoding the entire peptide of rat KiSS-1.

[SEQ ID NO:24]

This shows the amino acid sequence of a novel mouse G protein-coupled receptor protein mOT7T175.

[SEQ ID NO:25]

This shows the DNA sequence encoding a novel mouse G protein-coupled receptor protein mOT7T175.

[SEQ ID NO:26]

This shows the nucleic acid sequence of primer 1 used in Example 3.

[SEQ ID NO:27]

This shows the nucleic acid sequence of primer 2 used in Example 3.

*Escherichia coli* transformant DH10B/pCMV-mKiSS-1 obtained in Example 1 was on deposit with the Ministry of Economy, Trade and Industry, Agency of Industrial Science and Technology, National Institute of Bioscience and Human Technology (NIBH), located at 1-1-3, Higashi, Tsukuba-shi, Ibaraki, Japan, as the Accession Number FERM BP-7003 on January 24, 2000 and with Institute for Fermentation (IFO), located at 2-17-85, Juso Honcho, Yodogawa-ku, Osaka-shi, Osaka, Japan, as the Accession Number IFO 16348 on December 16, 1999.

*Escherichia coli* transformant DH5α/pCR2.1-mKiSS-1.4A obtained in Example 1 was on deposit with the Ministry of Economy, Trade and Industry, Agency of Industrial Science and Technology, National Institute of Bioscience and Human Technology (NIBH), located at 1-1-3, Higashi, Tsukuba-shi, Ibaraki, Japan, as the Accession Number FERM BP-7073 on March 6, 2000 and with Institute for Fermentation (IFO), located at 2-17-85, Juso Honcho, Yodogawa-ku, Osaka-shi, Osaka, Japan, as the Accession Number IFO 16360 on February 16, 2000.

*Escherichia coli* transformant TOP10/pRKISS4 obtained in Example 2 was on deposit with the Ministry of Economy, Trade and Industry, Agency of Industrial Science and Technology, National Institute of Bioscience and Human Technology (NIBH), located at 1-1-3, Higashi, Tsukuba-shi, Ibaraki, Japan, as the Accession Number FERM BP-7093 on March 16, 2000 and with Institute for Fermentation (IFO), located at 2-17-85, Juso Honcho, Yodogawa-ku, Osaka-shi, Osaka, Japan, as the Accession Number IFO 16362 on February 2, 2000.

*Escherichia coli* transformant DH5α/pCR-BluntII-mOT7T175 obtained in Example 3 was on deposit with the Ministry of Economy, Trade and Industry, Agency of Industrial Science and Technology, National Institute of Bioscience and Human Technology (NIBH), located at 1-1-3, Higashi, Tsukuba-shi, Ibaraki, Japan, as the Accession Number FERM BP-7428 on January 11, 2001 and with Institute for Fermentation (IFO), located at 2-17-85, Juso Honcho, Yodogawa-ku, Osaka-shi, Osaka, Japan, as the Accession Number IFO 16523 on December 22, 2000.

The present invention is described in detail below with reference to Examples, but is not deemed to limit the scope of the present invention thereto. The gene manipulation procedures using *Escherichia coli* were performed as described in the Molecular Cloning.

**Example 1**

Cloning of mouse KiSS-1

cDNA cloning was performed according to the manufacturer's instruction of GENE TRAPPER (Life Technologies). Probe A (SEQ ID NO: 9) was biotinylated and then hybridized with single-stranded mouse embryo cDNA library (Superscript cDNA library, Life Technologies) to obtain a single-stranded gene, which was then converted to double-stranded gene using primer B (SEQ ID NO. 10). This gene was introduced into Escherichia coli DH108 by electroporation, and the transformants were selected for ampicillin-resistance. Electroporation was performed using E. coli Pulser (BIO RAD) at a voltage of 1.8 kV. Transformants thus obtained were subjected to screening by colony PCR using primer B (SEQ ID NO. 10) and primer C (SEQ ID NO. 11), and a transformant (Escherichia coli) DH108/pCMV-mKiSS-1 was obtained. The ORF (open reading frame) (SEQ ID NO. 4) was deduced from the nucleotide sequence of this cDNA clone, and a novel secretory protein having the amino acid sequence (SEQ ID NO. 1) was designated mKiSS-1 (mouse type I).

For colony PCR, the reaction was carried out in the volume of 10 μl containing 1/50 volume of Advantage2 cDNA polymerase Mix (CLONTECH), 0.2 μM each of primer B (SEQ ID NO. 10) and primer C (SEQ ID NO. 11), 200 μM dNTPs, 1/25 volume of DMSO and the buffer attached to the enzyme product. The PCR was carried out (i) for 10 minutes at 94°C, followed by (ii) 25 cycles of 94°C for 10 seconds, 60°C for 10 seconds and 68°C for 1 minutes.

Then, primers D (SEQ ID NO. 12) and E (SEQ ID NO. 13), corresponding to the outside sequences of the ORF, were generated based on the above sequence of pCMV-mKiSS-1, and PCR was performed using mouse embryo Marathon Ready cDNA (CLONETECH) as a template. The PCR was performed in the reaction volume of 25 μl containing 1/50 volume of Advantage2 cDNA polymerase Mix (CLONTECH), 0.2 μM each of primer D (SEQ ID NO. 12) and primer E (SEQ ID NO. 13), 200 μM dNTPs, 1/25 volume of DMSO and the buffer attached to the enzyme product, with the following conditions: (i) for 2 minutes at 94°C, followed by (ii) 3 cycles of 94°C for 10 seconds and 68°C for 1 minute and 30 seconds, (iii) 3 cycles of 94°C for 10 seconds, 64°C for 10seconds and 68°C for 1 minute, then (iv) 30 cycles of 94°C for 10 seconds and 60°C for 10 seconds and 68°C for 1 minute, and finally (v) 68°C for 8 minutes for extension. The products obtained from the PCR were subcloned into plasmid vector pCR2.1-TOPO (Invitrogen), according to the manufacturer's instruction of the TOPO-TA cloning kit (Invitrogen), and then introduced into E. coli DH5α. The clones harboring the cDNA were selected on LB-agar medium containing ampicillin. Analysis of the sequence of each clone revealed that two types of clones were obtained: (i) a clone, nucleotide sequence of which is completely identical to that of mKiSS-1 described above (SEQ ID NO. 4), and (ii) a clone having 12 nucleotide insertion in the nucleotide sequence of mKiSS-1 described above (SEQ ID NO. 4) and one-nucleotide mutation (no amino acid change) at nucleotide position 402 from the starting position of the ORF. The 12-nucleotide insertion of (ii) can be translated into four amino acids, and both human KiSS-1 and rat KiSS-1 contain an amino acid sequence corresponding to the sequence of the four amino acids. Therefore, we considered that a mouse has two types of mKiSS-1, and designated the novel secretory protein having the amino acid sequence (SEQ ID NO. 2) deduced from the newly obtained nucleotide sequence (SEQ ID NO. 5) as mKiSS-1·4A (mouse type 2). Escherichia coli was transformed with the clone having the nucleotide sequence to obtain a transformant: Escherichia coli DH5α/pCR2.1-mKiSS-1.4A.

Amino acid sequences of mouse type 1 and type 2 KiSS-1 proteins (SEQ ID NOs: 4 and 5, respectively) deduced from the nucleotide sequences thus obtained showed high homology to the known human homolog (see Fig. 1).

Probe A

5'-tat ggg gag ccg ctg gca aaa gtg-3'

Primer B

5'-tag acc tgc ccc ttc ctc cca ga-3'

Primer C

5'-ctg ctg gcc tgt gga tcc agg ctt-3'

Primer D

5'-tgc agg aga gtg aag att aaa tcc cca-3'

Primer E

5'-gag gac ctg tcc cat ctc gca gga gtc a-3'

**Example 2**

Cloning of cDNA that encodes rat ligand (1-54) [rat KiSS-1]

Total RNA was extracted from rat placenta using TRIZOL reagent (Gibco BRL) according to the method described in the manufacturer's instruction. Next, poly(A)$^+$RNA was prepared from the total RNA using olig-dT cellulose column (MessageMaker reagent assembly, Gibco BRL) in accordance with the method described in the manufacturer's instruction. Subsequently, first strand cDNA was synthesized from the poly(A)$^+$RNA using Superscript Preamplification System for First Strand cDNA Synthesis (Gibco BRL) in accordance with the method described in the manufacturer's instruction. The degenerate primers as shown below were designed based on the mouse KiSS-1 sequence and synthesized:

13-3F38: 5'-TTCTTGGCAGCTRCTGCTTYTCCTCTGTG-3' (SEQ ID NO. 14)

KiSS357R: 5'-GAAGCGCAGGCCGAAGGAGTTCCA-3' (SEQ ID NO. 15)

Degenerative PCR was performed using the above-mentioned first strand cDNA as a template, and 13-3F38 and KiSS357R as primers. The reaction solution for the PCR was prepared by mixing 1 μl of Taq polymerase (TAKARA SHUZO), 10 μl of 10x PCR buffer (500 mM KCl, 100mM Tris·HCl, pH 8.3), 6 μl of 25 mM $MgCl_2$, 8 μl of 2.5 mM dNTP mixture, 4 μl of 25% DMSO solution, 2 μl each of primers 13-3F38 and KiSS357R (20 μM each), 2 μl of the template cDNA (the first strand cDNA described above) and 65 μl of distilled water. The PCR was performed with the following conditions to obtain PCR products: (1) initial denaturation (94°C for 5 minutes), (2) cycle reaction (94°C for 20 seconds-72°C for 50 seconds), (3) cycle reaction (94°C for 20 seconds-71.5°C for 20 seconds-72°C for 30 seconds), (4) cycle reaction (94°C for 20 seconds-71°C for 20 seconds-72°C for 30 seconds), (5) cycle reaction (94°C for 20 seconds-70.5°C for 20 seconds-72°C for 30 seconds), (6) cycle reaction (94°C for 20 seconds-72°C for 20 seconds-72°C for 30 seconds), (7) cycle reaction (94°C for 20 seconds-69.5°C for 20 seconds-72°C for 30 seconds), (8) cycle reaction (94°C for 20 seconds-69°C for 20 seconds-72°C for 30 seconds), (9) cycle reaction (94°C for 20 seconds-68.5°C for 20 seconds-72°C for 30 seconds), (10) cycle reaction (94°C for 20 seconds-68°C for 20 seconds-72°C for 30 seconds), (11) 30-cycle reaction (94°C for 20 seconds-61.8°C for 20 seconds-72°C for 30 seconds), followed by (12) final extension (72°C for 7 minutes).

The PCR products were then electrophoresed on 1.5% agarose gel, and a gel piece containing a cyber green-stained band of about 300 bp was cut out. A DNA fragment as the PCR product was recovered from the gel piece using the Gene Clean DNA extraction kit (BIO 101). Reaction for determination of nucleotide sequence of the DNA fragment was conducted using the Dye Terminator Cycle Sequencing Kit (Applied Biosystems, Perkin-Elmer), and the nucleotide sequence of the PCR product was determined using the automated fluorescence sequencer (DNA sequencer Prism 377: Applied Biosystems, Perkin-Elmer). As a result, the DNA fragment having the sequence encoding a partial peptide of rat KiSS-1 was obtained. The following sequence is a nucleotide sequence of pCR288bp (SEQ ID NO. 16) consisting of 288 bp without the degenerate primer portion. pCR288bp:

```
TGGCCTCTTT TGGGGAGCCA CTGGCAAAAA TGGCACCTGT GGTGAACCCT GAACCCACAG  60
GCCAACAGTC CGGACCCCAG GAACTCGTTA ATGCCTGGCA AAAGGGCCCG CGGTATGCAG 120
AGAGCAAGCC TGGGGCTGCA GGACTGCGCG CTCGCCGAAC ATCGCCATGC CCGCCGGTGG 180
AGAACCCCAC GGGGCACCAG CGGCCCCCGT GTGCCACCCG CAGTCGCCTG ATCCCTGCGC 240
CCCGCGGATC GGTGCTGGTG CAGCGCGAGA AGGACATGTC AGCCTACA             288
(SEQ ID NO. 16)
```

From the sequence thus obtained, primers 288-41F (SEQ ID NO. 17), 288-10F (SEQ ID NO. 18), 288-254R (SEQ ID NO. 19) and 288-44R (SEQ ID NO. 20) were prepared and used for the 5'-RACE and 3'-RACE experiments as described below.

288-41F: 5'-GGTGAACCCTGAACCCACAGGCCAACAG-3' (SEQ ID NO. 17)

288-10F: 5'-TTGGGGAGCCACTGGCAAAAATGGCACC-3' (SEQ ID NO. 18)

288-254R: 5'-TGACATGTCCTTCTCGCGCTGCACCAGC-3' (SEQ ID NO. 19)

288-44R: 5'-GGACTGTTGGCCTGTGGGTTCAGGGTTC-3' (SEQ ID NO. 20)

5'-RACE and 3'-RACE reactions were carried out using rat liver cDNA as a template. The reaction solution for the PCR for 5'-RACE and 3'-RACE was prepared by mixing 0.5 μl of Taq polymerase (TAKARA SHUZO), 5 μl of 10x PCR buffer attached (500 mM KCl, 25 mM $MgCl_2$, 100mM Tris·HCl, pH 8.3), 4 μl of 2.5 mM dNTP mixture, 3 μl of 25 mM $MgCl_2$, 2 μl of 25% DMSO solution (for 3'-RACE), 1 μl of 10 μM primer 288-10F (for 3'-RACE) or 10 μM primer 288-254R (for 5'-RACE) , 1 μl of 10 μM primer AP1 (primer AP1 was included in the Marathon-Ready cDNA Kit supplied by CLONTECH), 5 μl of template rat liver cDNA (Marathon-Ready cDNA Kit, rat liver, CLONTECH) and 28.5 μl (for 3'-RACE) or 30.5 μl (for 5'-RACE)of distilled water. The conditions for the reaction were as follows: 94°C for 60 seconds for initial denaturation, followed by 5 cycles of 94°C for 30 seconds and 72°C for 120 seconds, 5 cycles of 94°C for 30 seconds and 70°C for 120 seconds, 25 cycles of 94°C for 20 seconds and 68°C for 120 seconds and final extension at 68°C for 10 minutes.

Subsequently, nested PCR was performed using the PCR product solution as a template. The reaction solution was prepared by mixing 0.5 μl of Taq polymerase (TAKARA SHUZO), 5 μl of 10x PCR buffer (500 mM KCl, 25 mM $MgCl_2$, 100mM Tris·HCl, pH 8.3), 4 μl of 2.5 mM dNTP mixture, 3 μl of 25 mM $MgCl_2$, 2 μl of 25% DMSO solution (for 3'-RACE), 1 μl of 10 μM primer 288-41F (for 3'-RACE) or 10 μM primer 288-44R (for 5'-RACE) , 1 μl of 10 μM primer AP2 (primer AP2 was included in the Marathon-Ready cDNA Kit supplied by CLONTECH), 5 μl of template DNA (50-fold dilution of the PCR product) and 28.5 μl (for 3'-RACE) or 30.5 μl (for 5'-RACE) of distilled water. The reaction conditions were as follows: initial denaturation at 94°C for 60 seconds, followed by 5 cycles of 94°C for 30 seconds and 72°C for 120 seconds, 5 cycles of 94°C for 30 seconds and 70°C for 120 seconds, 25 cycles of 94°C for 20 seconds and 68°C for 120 seconds and final extension at 68°C for 10 minutes.

From the nested PCR product, a DNA fragment was recovered according to the method as described above. The DNA fragment were ligated to plasmid vector pCR2.1 using the TOPO-TA Cloning Kit (Invitrogen) in accordance with the manufacturer's instruction, and used to transform E. coli TOP10. E. coli was picked up from a single colony and liquid-cultured in LB medium. After the culture, the cell was collected and the plasmid was purified using a plasmid purification kit (QIAwell 8 Ultra plasmid purification kit: QIAGEN). The nucleotide sequence of the PCR product inserted into the plasmid was analyzed according to the method as described above to obtain sequence information of the 5'- and 3' ends. Based on this information, primers rKiSS364F and rKiSS859R were prepared.

rKiSS364F: 5'-CGTCTCAGCCTCTGGACACCCTGTGGATCTGCC-3'(SEQ ID NO. 21)

rKiSS859R: 5'-TGGCGACAGCATTGCTTTTATTGCACAAGTCTA-3'(SEQ ID NO. 22)

PCR was performed with primers rKiSS364F and rKiSS859R, using rat liver cDNA as a template. The reaction solution for the PCR was prepared by mixing 1 µl of Pfu DNA polymerase (Stratagene), 5 µl of 10x PCR buffer (500 mM KCl, 25 mM $MgCl_2$, 100mM Tris·HCl, pH 8.3), 4 µl of 2.5 mM dNTP mixture, 2 µl of 25% DMSO solution, 1 µl each of 10 µM primers rKiSS364F and rKiSS859R , 5 µl of template rat liver cDNA (Marathon-Ready cDNA Kit, rat liver, CLONTECH) and 31 µl of distilled water. The conditions for the reaction were as follows: initial denaturation at 94°C for 60 seconds, followed by 3 cycles of 94°C for 20 seconds and 72°C for 120 seconds, 3 cycles of 94°C for 20 seconds and 70°C for 120 seconds, 3 cycles of 94°C for 20 seconds and 68°C for 120 seconds, 30 cycles of 94°C for 20 seconds, 63°C for 30 seconds and 68°C for 120 seconds, and final extension at 68°C for 10 minutes. The DNA fragment thus obtained was cloned into pPCR-BluntII-TOPO vector (Stratagene) according to the method provided in the manufacturer's instruction. The cloned DNA sequence was analyzed according to the method as described above, and pRKISS4, which contains a 393 bp DNA fragment encoding the complete peptide of rat KiSS-1, was successfully obtained. E. coli TOP10 transformed with the plasmid was designated TOP10/pRKISS4.

The amino acid sequence of the rat KiSS-1 protein (SEQ ID NO. 6) deduced from the nucleotide sequence obtained showed high homology with its known human homolog (see Fig. 1).

pRKISS4(393 bp):

```
ATGACCTCGC TGGCTTCTTG GCAGCTGCTG CTTCTCCTCT GTGTGGCCTC TTTTGGGGAG  60
CCACTGGCAA AAATGGCACC TGTGGTGAAC CCTGAACCCA CAGGCCAACA GTCCGGACCC 120
CAGGAACTCG TTAATGCCTG GCAAAAGGGC CCGCGGTATG CAGAGAGCAA GCCTGGGGCT 180
GCAGGACTGC GCGCTCGCCG AACATCGCCA TGCCCGCCGG TGGAGAACCC CACGGGGCAC 240
CAGCGGCCCC CGTGTGCCAC CCGCAGTCGC CTGATCCCTG CGCCCCGCGG ATCGGTGCTG 300
GTGCAGCGCG AGAAGGACAT GTCAGCCTAC AACTGGAACT CCTTTGGCCT GCGCTACGGC 360
AGGAGGCAGG TGGCGCGGGC GGCACGGGGC TGA                             393
(SEQ ID NO. 23)
```

## Example 3

Cloning and sequencing of cDNA encoding a novel G-protein coupled receptor protein derived from mouse whole brain

PCR was performed using mouse whole brain cDNA (CLONTECH) as a template and two primers: primer 1 (SEQ ID NO. 26) and primer 2 (SEQ ID NO. 27). The composition of the reaction solution used in the reaction was as follows: 1/10 volume of the above cDNA was used as a template, and mixed with 1/50 volume of Pfu Turbo DNA Polymerase (STRATAGENE), 0.2 µM each of primer 1 and primer 2, 200 µM dNTPs, and the buffer attached to the enzyme product. The volume of the reaction solution was adjusted to 25 µl. The PCR reaction was carried out as follows: (i) 94°C for 2 minutes, followed by (ii) 3 cycles of 94°C for 20 seconds and 72°C for 2 minutes, (iii) 3 cycles of 94°C for 20 seconds and 68°C for 2 minutes, (iv) 38 cycles of 94°C for 20 seconds, 62°C for 20 seconds and 68°C for 1 minute and 30 seconds, and final extension at 68°C for 7 minutes. The product obtained from the PCR were subcloned into a plasmid vector pCR-Blunt II-TOPO (Invitrogen), according to the manufacturer's instruction for the Zero-blunt TOPO-TA Cloning Kit (Invitrogen). The plasmid was then introduced into E. coli DH5α and clones harboring the cDNA were selected on LB-agar medium containing kanamycin. As the result of analyzing the sequence of each clone, a cDNA sequence encoding a novel G-protein coupled receptor protein (SEQ ID NO. 25) was obtained. The amino acid sequence deduced from the nucleotide sequence consisted of 396 residues (SEQ ID NO. 24) and showed the highest homology of 94.4% with a known G-protein coupled receptor protein rOT7T175 (GPR54) and 82.4% homology with its human counterpart, hOT7T175 (WO 00/24890). Therefore, the amino acid sequence was considered to be a mouse counterpart of these proteins. Thus, the novel G-protein coupled receptor protein comprising the amino acid sequence was designated mOT7T175. The transformant harboring the sequence encoding mOT7T175 was designated Escherichia coli DH5α/pCR-Blund II-mOT7T175.

## INDUSTRIAL APPLICABILITY

The present invention provides novel ligand proteins derived from rats and mice and partial peptides thereof, or salts thereof, and polynucleotides (e.g. DNA, RNA, and derivatives thereof) encoding them. Also provided are vectors containing any of said polynucleotides, a method for producing said ligand proteins using any of said vectors. Further provided are antibodies and antagonists against said proteins and the like, and a method and kit of screening for them . Pharmaceuticals and the like that comprise any of said proteins, polynucleotides or antibodies or antagonist against them are also provided.

## Claims

1. A protein comprising the same or substantially the same amino acid sequence as that represented by SEQ ID NO: 1 or NO:3, or a salt thereof.

2. The protein or salt thereof according to claim 1, wherein substantially the same amino acid sequence is the sequence represented by SEQ ID NO:2.

3. A partial peptide of the protein according to claim 1, which comprises the amino acid sequence of from the 132 to 141th residues from the N terminal of the amino acid sequence represented by SEQ ID NO:1, or a salt thereof.

4. The partial peptide or salt thereof according to claim 3, which comprises the same or substantially the same amino acid sequence as that of from the 127 to 141th residues from the N terminal of the amino acid sequence represented by SEQ ID NO:1.

5. The partial peptide or salt thereof according to claim 3, which has the same or substantially the same amino acid sequence as that of from the 90 to 141th residues from the N terminal of the amino acid sequence represented by SEQ ID NO:1.

**6.** A partial peptide of the protein according to claim 1, which has the same or substantially the same amino acid sequence as that of from the 94 to 145th residues from the N terminal of the amino acid sequence represented by SEQ ID NO:2, or a salt thereof.

**7.** A partial peptide of the protein according to claim 1, which comprises the amino acid sequence of from the 110 to 119th residues from the N terminal of the amino acid sequence represented by SEQ ID NO:3, or a salt thereof.

**8.** The partial peptide or salt thereof according to claim 7, which comprises the same or substantially the same amino acid sequence as that of from the 105 to 119th residues from the N terminal of the amino acid sequence represented by SEQ ID NO:3.

**9.** The partial peptide or salt thereof according to claim 7, which has the same or substantially the same amino acid sequence as that of from the 68 to 119th residues from the N terminal of the amino acid sequence represented by SEQ ID NO:3.

**10.** A polynucleotide comprising a polynucleotide encoding the protein according to claim 1.

**11.** A polynucleotide comprising a polynucleotide encoding the partial peptide according to claim 3, 6 or 7.

**12.** The polynucleotide according to claim 10 or 11, which is DNA.

**13.** The polynucleotide according to claim 10, which has the nucleic acid sequence represented by SEQ ID NO:4, NO:5 or NO:6.

**14.** A recombinant vector comprising the polynucleotide according to claim 10 or 11.

**15.** A transformant transformed with the recombinant vector according to claim 14.

**16.** A method of producing the protein or salt thereof according to claim 1, or the partial peptide or salt thereof according to claim 3, 6 or 7, which comprises culturing the transformant according to claim 15, and making it produce and accumulate the protein according to claim 1, or the partial peptide according to claim 3, 6 or 7.

**17.** An antibody to the protein or salt thereof according to claim 1, or the partial peptide or salt thereof according to claim 3, 6 or 7.

**18.** The antibody according to claim 17, which is a neutralizing antibody capable of inactivating signal transduction of the protein according to claim 1 or the partial peptide according to claim 3, 6 or 7.

**19.** A method of screening a compound or salt thereof which alters the binding property between a receptor and the protein or salt thereof according to claim 1 or the partial peptide or salt thereof according to claim 3, 6 or 7, which comprises using the protein or salt thereof according to claim 1 or the partial peptide or salt thereof according to claim 3, 6 or 7.

**20.** A kit for screening a compound or salt thereof which alters the binding property between a receptor and the protein or salt thereof according to claim 1 or the partial peptide or salt thereof according to claim 3, 6 or 7, which comprises the protein or salt thereof according to claim 1 or the partial peptide or salt thereof according to claim 3, 6 or 7.

**21.** The screening method according to claim 19 or the screening kit according to claim 20, wherein the receptor is a protein or salt thereof having the same or substantially the same amino acid sequence as that represented by SEQ ID NO:7, NO:8 or NO:24.

**22.** A compound or salt thereof which alters the binding property between a receptor and the protein or salt thereof according to any one of claims 1, 3, 4 and 5, which is obtainable using the screening method according to claim 19 or the screening kit according to claim 20.

**23.** The compound or salt thereof according to claim 22, which is an agonist.

**24.** A pharmaceutical composition comprising a compound or salt thereof which alters the binding property between a receptor and the protein or salt thereof according to claim 1 or the partial peptide or salt thereof according to claim 3, 6 or 7, which is obtainable using the screening method according to claim 19 or the screening kit according to claim 20.

**25.** The pharmaceutical composition according to claim 24, which is an agent for inhibiting tumor metastasis.

**26.** A method of quantifying the protein according to claim 1 or the partial peptide according to claim 3, 6 or 7, which comprises using the antibody according to claim 17.

**27.** A pharmaceutical composition comprising the protein or salt thereof according to claim 1 or the partial peptide or salt thereof according to claim 3, 6 or 7.

**28.** The pharmaceutical composition according to claim 27, which is an agent for inhibiting tumor metastasis.

# Fig. 1

KISS-1 sequence comparsion

```
human            1:--------------------------MNSLVSWQLLLFLCATHFGEPLEKVASVGNSRPT 34
mouse type 1     1:MYLRFGVDVCSLSPWKETVDLPLPPRMISMASWQLLLLLCVATYGEPLAK----VKPGST 56
mouse type 2     1:MYLRFGVDVCSLSPWKETVDLPLPPRMISMASWQLLLLLCVATYGEPLAKVAPLVKPGST 60
rat              1:--------------------------MISLASWQLLLLLCVASFGEPLAKMAPVVNPEPT 34
                                             * *  ****** **    **** *         *

human           35:GQQLESLGLLAPGEQSLPCTERKPAATARLSRRGTSLSPPPESSGSRQQPGLSAPHSRQI 94
mouse type 1    57:GQQSGPQELVNAWEKESRYAESKPGSAGLRARRSSP-CPPVEGPAGRQRP-LCASRSRLI 114
mouse type 2    61:GQQSGPQELVNAWEKESRYAESKPGSAGLRARRSSP-CPPVEGPAGRQRP-LCASRSRLI 118
rat             35:GQQSGPQELVNAWQKGPRYAESKPGAAGLRARRTSP-CPPVENPTGHQRP-PCATRSRLI 92
                   ***     *           * **       **     ** *     * *   *  ** *

human           95:PAPQGAVLVQREKDLPNYNWNSFGLRFGKREAAPGNHGRSAGRGWGAGAGQ          145
mouse type 1   115:PAPRGAVLVQREKDLSTYNWNSFGLRYGRRQAARAARG-------------          152
mouse type 2   119:PAPRGAVLVQREKDLSTYNWNSFGLRYGRRQAARAARG-------------          156
rat             93:PAPRGSVLVQREKDMSAYNWNSFGLRYGRRQVARAARG-------------          130
                   *** * ********   ********* * *  *    *
```

**INTERNATIONAL SEARCH REPORT**

International application No.
PCT/JP01/02615

A. CLASSIFICATION OF SUBJECT MATTER
Int.Cl[7] C12N 15/12, 1/21, C07K 14/47, 16/18, C12P 21/02, G01N 33/53, A61K 38/00, 45/00, 48/00, A61P 35/04

According to International Patent Classification (IPC) or to both national classification and IPC

B. FIELDS SEARCHED

Minimum documentation searched (classification system followed by classification symbols)
Int.Cl[7] C12N 15/00-15/90, C07K 14/00-14/825

Documentation searched other than minimum documentation to the extent that such documents are included in the fields searched

Electronic data base consulted during the international search (name of data base and, where practicable, search terms used)
MEDLINE (STN), WPI (DIALOG), BIOSIS (DIALOG), JICST FILE (JOIS)
GenBank/EMBL/DDBJ/GeneSeq, SwissProt/PIR/GeneSeq

C. DOCUMENTS CONSIDERED TO BE RELEVANT

| Category* | Citation of document, with indication, where appropriate, of the relevant passages | Relevant to claim No. |
|---|---|---|
| P,X | WO, 00/24890, A1 (Takeda Chemical Industries, Ltd.), 04 May, 2000 (04.05.00), & AU, 9962306, A | 1-28 |
| X<br>A | Maria de Fatima Bonaldo et al., "Normalization and Subtraction: Two Approaches to Facilitate Gene Discovery", Genome Res. (1996), Vol.6, No.9, pages 791 to 806 | 10,12,13<br>1-9,11,14-28 |
| X<br>A | Ande West et al., "Chromosome Localization and Genomic Structure of the KiSS-1 Metastasis Suppressor Gene(KISS1)",Genomics (1998), Vol.54, pages 145 to 148 | 1-18,26-28<br>19-25 |
| P,A | WO, 00/50563, A2 (Merck & Co., Inc.), 31 August, 2000 (31.08.00) (Family: none) | 1-28 |
| A | WO, 98/03548, A1 (Astra Pharma Inc.), 29 January, 1998 (29.01.98), & AU, 9737115, A & EP, 912610, A1 | 1-28 |

☒ Further documents are listed in the continuation of Box C. ☐ See patent family annex.

* *Special categories of cited documents:*
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" *later document published after the international filing date or* priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 June, 2001 (14.06.01) | 26 June, 2001 (26.06.01) |
| Name and mailing address of the ISA/<br>Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/02615

C (Continuation). DOCUMENTS CONSIDERED TO BE RELEVANT

| Category* | Citation of document, with indication, where appropriate, of the relevant passages | Relevant to claim No. |
|---|---|---|
| T | Tetsuya OHTAKI et al., "Metastasis suppressor gene *KiSS-1* encodes peptide ligand of a G-protein-coupled receptor", Nature, (May, 2001), Vol.411, pages 613 to 617 | 1-28 |

Form PCT/ISA/210 (continuation of second sheet) (July 1992)

SEQUENCE LISTING

<110> Takeda Chemical Industries, Ltd.

<120> Novel protein, DNA encoding the same, and process for preparation thereof

<130> 662503

<150> JP 2000-093575

<151> 2000-03-30

<160> 27

<210> 1

<211> 152

<212> PRT

<213> Mouse

<400> 1

Met Tyr Leu Arg Phe Gly Val Asp Val Cys Ser Leu Ser Pro Trp Lys
5 10 15

Glu Thr Val Asp Leu Pro Leu Pro Pro Arg Met Ile Ser Met Ala Ser
20 25 30

Trp Gln Leu Leu Leu Leu Leu Cys Val Ala Thr Tyr Gly Glu Pro Leu
35 40 45

Ala Lys Val Ala Pro Gly Ser Thr Gly Gln Gln Ser Gly Pro Gln Glu
50 55 60

Leu Val Asn Ala Trp Glu Lys Glu Ser Arg Tyr Ala Glu Ser Lys Pro
65 70 75 80

Gly Ser Ala Gly Leu Arg Ala Arg Arg Ser Ser Pro Cys Pro Pro Val
85 90 95

Glu Gly Pro Ala Gly Arg Gln Arg Pro Leu Cys Ala Ser Arg Ser Arg
100 105 110

Leu Ile Pro Ala Pro Arg Gly Ala Val Leu Val Gln Arg Glu Lys Asp

115 120 125

Leu Ser Thr Tyr Asn Trp Asn Ser Phe Gly Leu Arg Tyr Gly Arg Arg

130 135 140

Gln Ala Ala Arg Ala Ala Arg Gly

145 150

<210> 2

<211> 156

<212> PRT

<213> Mouse

<400> 2

Met Tyr Leu Arg Phe Gly Val Asp Val Cys Ser Leu Ser Pro Trp Lys

5 10 15

Glu Thr Val Asp Leu Pro Leu Pro Pro Arg Met Ile Ser Met Ala Ser

20 25 30

Trp Gln Leu Leu Leu Leu Leu Cys Val Ala Thr Tyr Gly Glu Pro Leu

35 40 45

Ala Lys Val Ala Pro Leu Val Lys Pro Gly Ser Thr Gly Gln Gln Ser

50 55 60

Gly Pro Gln Glu Leu Val Asn Ala Trp Glu Lys Glu Ser Arg Tyr Ala

65 70 75 80

Glu Ser Lys Pro Gly Ser Ala Gly Leu Arg Ala Arg Arg Ser Ser Pro

85 90 95

Cys Pro Pro Val Glu Gly Pro Ala Gly Arg Gln Arg Pro Leu Cys Ala

100 105 110

Ser Arg Ser Arg Leu Ile Pro Ala Pro Arg Gly Ala Val Leu Val Gln

115 120 125

Arg Glu Lys Asp Leu Ser Thr Tyr Asn Trp Asn Ser Phe Gly Leu Arg

130 135 140

Tyr Gly Arg Arg Gln Ala Ala Arg Ala Ala Arg Gly

145 150 155

<210> 3

<211> 130

<212> PRT

<213> Rat

<400> 3

Met Thr Ser Leu Ala Ser Trp Gln Leu Leu Leu Leu Leu Cys Val Ala
5 10 15

Ser Phe Gly Glu Pro Leu Ala Lys Met Ala Pro Val Val Asn Pro Glu
20 25 30

Pro Thr Gly Gln Gln Ser Gly Pro Gln Glu Leu Val Asn Ala Trp Gln
35 40 45

Lys Gly Pro Arg Tyr Ala Glu Ser Lys Pro Gly Ala Ala Gly Leu Arg
50 55 60

Ala Arg Arg Thr Ser Pro Cys Pro Pro Val Glu Asn Pro Thr Gly His
65 70 75 80

Gln Arg Pro Pro Cys Ala Thr Arg Ser Arg Leu Ile Pro Ala Pro Arg
85 90 95

Gly Ser Val Leu Val Gln Arg Glu Lys Asp Met Ser Ala Tyr Asn Trp
100 105 110

Asn Ser Phe Gly Leu Arg Tyr Gly Arg Arg Gln Val Ala Arg Ala Ala
115 120 125

Arg Gly
130

<210> 4

<211> 449

<212> DNA

<213> Mouse

<400> 4

atgtatctga gatttggcgt tgatgtctgc agcctgagtc cctggaagga gactgtagac 60

ctgccccttc ctcccagaat tctcaatggc ttcttggcag ctgctgcttc tcctctgtgt 120

cgccacctat ggggagccgc tggcaaaagt gaagcctgga cacaggccag cagtccggac 180

cccaggaact cgttaatgcc tgggaaaagg aatcgcggta tgcagagagc aagcctgggt 240

gcagggctgc gcgctcgtag gtcgtcgcca tgcccgccgg ttgagggccc cgcggggcgc 300

cagcggcccc tgtgtgcctc gcagtcgcct gatccctgcg ccccgcggag cggtgctggt 360

gcagcgggag aaggacctgt ccacctacaa ctggaactcc cggcctgcgc tacggcagga 420

ggcaggcggc gcgggcagca cggggctga 449

<210> 5

<211> 458

<212> DNA

<213> Mouse

<400> 5

atgtatctga gatttggcgt tgatgtctgc agcctgagtc cctggaagga gactgtagac 60

ctgccccttc ctcccagaat tctcaatggc ttcttggcag ctgctgcttc tcctctgtgt 120

cgccacctat ggggagccgc tggcaaaagt ggcacctttg gaagcctgga tccacaggcc 180

agcagtccgg accccaggaa ctcgttaatg cctgggaaaa ggaatcgcgg tatgcagaga 240

aagcctgggt ctgcagggct gcgcgctcgt aggtcgtcgc catgcccgcc ggttgagggc 300

cccgcggggc gccagcggcc tgtgtgcctc ccgcagtcgc ctgatccctg cgccccgcgg 360

agcggtgctg gtgcagcggg agaaggacct gtcgacctac ctggaactcc ttcggcctgc 420

gctacggcag gaggcaggcg gcgcgggcag cacggggc 458

<210> 6

<211> 390

<212> DNA

<213> Rat

<400> 6

atgacctcgc tggcttcttg gcagctgctg cttctcctct gtgtggcctc ttttggggag 60

ccactggcaa aaatggcacc tgtggtgaac cctgaaccca caggccaaca gtccggaccc 120

caggaactcg ttaatgcctg gcaaaagggc ccgcggtatg cagagagcaa gcctggggct 180

gcaggactgc gcgctcgccg aacatcgcca tgcccgccgg tggagaaccc cacggggcac 240

cagcggcccc cgtgtgccac ccgcagtcgc ctgatccctg cgcccgcgg atcggtgctg 300

gtgcagcgcg agaaggacat gtcagcctac aactggaact cctttggcct gcgctacggc 360

aggaggcagg tggcgcgggc ggcacggggc 390

<210> 7

<211> 398

<212> PRT

<213> Human

<400> 7

Met His Thr Val Ala Thr Ser Gly Pro Asn Ala Ser Trp Gly Ala Pro
5 10 15

Ala Asn Ala Ser Gly Cys Pro Gly Cys Gly Ala Asn Ala Ser Asp Gly
20 25 30

Pro Val Pro Ser Pro Arg Ala Val Asp Ala Trp Leu Val Pro Leu Phe
35 40 45

Phe Ala Ala Leu Met Leu Leu Gly Leu Val Gly Asn Ser Leu Val Ile
50 55 60

Tyr Val Ile Cys Arg His Lys Pro Met Arg Thr Val Thr Asn Phe Tyr
65 70 75 80

Ile Ala Asn Leu Ala Ala Thr Asp Val Thr Phe Leu Leu Cys Cys Val
85 90 95

Pro Phe Thr Ala Leu Leu Tyr Pro Leu Pro Gly Trp Val Leu Gly Asp
100 105 110

Phe Met Cys Lys Phe Val Asn Tyr Ile Gln Gln Val Ser Val Gln Ala
115 120 125

Thr Cys Ala Thr Leu Thr Ala Met Ser Val Asp Arg Trp Tyr Val Thr
130 135 140

Val Phe Pro Leu Arg Ala Leu His Arg Arg Thr Pro Arg Leu Ala Leu
145 150 155 160

Ala Val Ser Leu Ser Ile Trp Val Gly Ser Ala Ala Val Ser Ala Pro
165 170 175

Val Leu Ala Leu His Arg Leu Ser Pro Gly Pro Arg Ala Tyr Cys Ser
180 185 190

Glu Ala Phe Pro Ser Arg Ala Leu Glu Arg Ala Phe Ala Leu Tyr Asn
195 200 205

Leu Leu Ala Leu Tyr Leu Leu Pro Leu Leu Ala Thr Cys Ala Cys Tyr
210 215 220

Ala Ala Met Leu Arg His Leu Gly Arg Val Ala Val Arg Pro Ala Pro
225 230 235 240

Ala Asp Ser Ala Leu Gln Gly Gln Val Leu Ala Glu Arg Ala Gly Ala
245 250 255

Val Arg Ala Lys Val Ser Arg Leu Val Ala Ala Val Val Leu Leu Phe
260 265 270

Ala Ala Cys Trp Gly Pro Ile Gln Leu Phe Leu Val Leu Gln Ala Leu
275 280 285

Gly Pro Ala Gly Ser Trp His Pro Arg Ser Tyr Ala Ala Tyr Ala Leu
290 295 300

Lys Thr Trp Ala His Cys Met Ser Tyr Ser Asn Ser Ala Leu Asn Pro
305 310 315 320

Leu Leu Tyr Ala Phe Leu Gly Ser His Phe Arg Gln Ala Phe Arg Arg
325 330 335

Val Cys Pro Cys Ala Pro Arg Arg Pro Arg Arg Pro Arg Arg Pro Gly
340 345 350

Pro Ser Asp Pro Ala Ala Pro His Ala Glu Leu His Arg Leu Gly Ser
355 360 365

His Pro Ala Pro Ala Arg Ala Gln Lys Pro Gly Ser Ser Gly Leu Ala
370 375 380

Ala Arg Gly Leu Cys Val Leu Gly Glu Asp Asn Ala Pro Leu

385 390 395

<210> 8

<211> 396

<212> PRT

<213> Rat

<400> 8

Met Ala Ala Glu Ala Thr Leu Gly Pro Asn Val Ser Trp Trp Ala Pro
5 10 15

Ser Asn Ala Ser Gly Cys Pro Gly Cys Gly Val Asn Ala Ser Asp Gly
20 25 30

Pro Gly Ser Ala Pro Arg Pro Leu Asp Ala Trp Leu Val Pro Leu Phe
35 40 45

Phe Ala Ala Leu Met Leu Leu Gly Leu Val Gly Asn Ser Leu Val Ile
50 55 60

Phe Val Ile Cys Arg His Lys His Met Gln Thr Val Thr Asn Phe Tyr
65 70 75 80

Ile Ala Asn Leu Ala Ala Thr Asp Val Thr Phe Leu Leu Cys Cys Val
85 90 95

Pro Phe Thr Ala Leu Leu Tyr Pro Leu Pro Thr Trp Val Leu Gly Asp
100 105 110

Phe Met Cys Lys Phe Val Asn Tyr Ile Gln Gln Val Ser Val Gln Ala
115 120 125

Thr Cys Ala Thr Leu Thr Ala Met Ser Val Asp Arg Trp Tyr Val Thr
130 135 140

Val Phe Pro Leu Arg Ala Leu His Arg Arg Thr Pro Arg Leu Ala Leu
145 150 155 160

Thr Val Ser Leu Ser Ile Trp Val Gly Ser Ala Ala Val Ser Ala Pro
165 170 175

Val Leu Ala Leu His Arg Leu Ser Pro Gly Pro His Thr Tyr Cys Ser

180 185 190

Glu Ala Phe Pro Ser Arg Ala Leu Glu Arg Ala Phe Ala Leu Tyr Asn

195 200 205

Leu Leu Ala Leu Tyr Leu Leu Pro Leu Leu Ala Thr Cys Ala Cys Tyr

210 215 220

Gly Ala Met Leu Arg His Leu Gly Arg Ala Ala Val Arg Pro Ala Pro

225 230 235 240

Thr Asp Gly Ala Leu Gln Gly Gln Leu Leu Ala Gln Arg Ala Gly Ala

245 250 255

Val Arg Thr Lys Val Ser Arg Leu Val Ala Ala Val Val Leu Leu Phe

260 265 270

Ala Ala Cys Trp Gly Pro Ile Gln Leu Phe Leu Val Leu Gln Ala Leu

275 280 285

Gly Pro Ser Gly Ala Trp His Pro Arg Ser Tyr Ala Ala Tyr Ala Leu

290 295 300

Lys Ile Trp Ala His Cys Met Ser Tyr Ser Asn Ser Ala Leu Asn Pro

305 310 315 320

Leu Leu Tyr Ala Phe Leu Gly Ser His Phe Arg Gln Ala Phe Cys Arg

325 330 335

Val Cys Pro Cys Gly Pro Gln Arg Gln Arg Arg Pro His Ala Ser Ala

340 345 350

His Ser Asp Arg Ala Ala Pro His Ser Val Pro His Ser Arg Ala Ala

355 360 365

His Pro Val Arg Val Arg Thr Pro Glu Pro Gly Asn Pro Val Val Arg

370 375 380

Ser Pro Ser Val Gln Asp Glu His Thr Ala Pro Leu

385 390 395

<210> 9

<211> 24

<212> DNA

<213> Artifical Sequence

<220>

<223> Designed oligonucleotide probe to amplify mouse KiSS-1 cDNA.

<400> 9

tatggggagc cgctggcaaa agtg 24

<210> 10

<211> 23

<212> DNA

<213> Artificial Sequence

<220>

<223> Designed oligonucleotide primer to amplify mouse KiSS-1 cDNA.

<400> 10

tagacctgcc ccttcctccc aga 23

<210> 11

<211> 24

<212> DNA

<213> Artificial Sequence

<220>

<223> Designed oligonucleotide primer to amplify mouse KiSS-1 cDNA.

<400> 11

ctgctggcct gtggatccag gctt 24

<210> 12

<211> 27

<212> DNA

<213> Artificial Sequence

<220>

<223> Designed oligonucleotide primer to amplify mouse KiSS-1 cDNA.

<400> 12

tgcaggagag tgaagattaa atcccca 27

<210> 13

<211> 28

<212> DNA

<213> Artificial Sequence

<220>

<223> Designed oligonucleotide primer to amplify mouse KiSS-1 cDNA.

<400> 13

gaggacctgt cccatctcgc aggagtca 28

<210> 14

<211> 29

<212> DNA

<213> Artificial Sequence

<220>

<223> Designed oligonucleotide primer to amplify rat KiSS-1 cDNA.

<400> 14

ttcttggcag ctrctgctty tcctctgtg 29

<210> 15

<211> 24

<212> DNA

<213> Artificial Sequence

<220>

<223> Designed oligonucleotide primer to amplify rat KiSS-1 cDNA.

<400> 15

gaagcgcagg ccgaaggagt tcca 24

<210> 16

<211> 288

<212> DNA

<213> Rat

<400> 16

tggcctcttt tggggagcca ctggcaaaaa tggcacctgt ggtgaaccct gaacccacag 60

gccaacagtc cggaccccag gaactcgtta atgcctggca aaagggcccg cggtatgcag 120

agagcaagcc tggggctgca ggactgcgcg ctcgccgaac atcgccatgc ccgccggtgg 180

agaaccccac ggggcaccag cggcccccgt gtgccacccg cagtcgcctg atccctgcgc 240

cccgcggatc ggtgctggtg cagcgcgaga aggacatgtc agcctaca 288

<210> 17

<211> 28

<212> DNA

<213> Artificial Sequence

<220>

<223> Designed oligonucleotide primer for 3'-RACE to amplify rat KiSS-1 cDNA.

<400> 17

ggtgaaccct gaacccacag gccaacag 28

<210> 18

<211>

<212> DNA

<213> Artificial Sequence

<220>

<223> Designed oligonucleotide primer to amplify rat KiSS-1 cDNA.

<400> 18

ttggggagcc actggcaaaa atggcacc 28

<210> 19

<211>

<212> DNA

<213> Artificial Sequence

<220>

<223> Designed oligonucleotide primer to amplify rat KiSS-1 cDNA.

<400> 19

tgacatgtcc ttctcgcgct gcaccagc 28

<210> 20

<211> 28

<212> DNA

<213> Artificial Sequence

<220>

<223> Designed oligonucleotide primer for 5'-RACE to amplify rat KiSS-1 cDNA.

<400> 20

ggactgttgg cctgtgggtt cagggttc 28

<210> 21

<211> 33

<212> DNA

<213> Artificial Sequence

<220>

<223> Designed oligonucleotide primer to amplify rat KiSS-1 cDNA.

<400> 21

cgtctcagcc tctggacacc ctgtggatct gcc 33

<210> 22

<211> 33

<212> DNA

<213> Artificial Sequence

<220>

<223> Designed oligonucleotide primer to amplify rat KiSS-1 cDNA.

<400> 22

tggcgacagc attgctttta ttgcacaagt cta 33

<210> 23

<211> 393

<212> DNA

<213> Rat

<400> 23

atgacctcgc tggcttcttg gcagctgctg cttctcctct gtgtggcctc ttttggggag 60

ccactggcaa aaatggcacc tgtggtgaac cctgaaccca caggccaaca gtccggaccc 120

caggaactcg ttaatgcctg gcaaaagggc ccgcggtatg cagagagcaa gcctggggct 180

gcaggactgc gcgctcgccg aacatcgcca tgcccgccgg tggagaaccc cacggggcac 240

cagcggcccc cgtgtgccac ccgcagtcgc ctgatccctg cgccccgcgg atcggtgctg 300

gtgcagcgcg agaaggacat gtcagcctac aactggaact cctttggcct gcgctacggc 360

aggaggcagg tggcgcgggc ggcacggggc tga 393

<210> 24

<211> 396

<212> PRT

<213> Mouse

<400> 24

Met Ala Thr Glu Ala Thr Leu Ala Pro Asn Val Thr Trp Trp Ala Pro

1 5 10 15

Ser Asn Ala Ser Gly Cys Pro Gly Cys Gly Val Asn Ala Ser Asp Asp

20 25 30

Pro Gly Ser Ala Pro Arg Pro Leu Asp Ala Trp Leu Val Pro Leu Phe

35 40 45

Phe Ala Thr Leu Met Leu Leu Gly Leu Val Gly Asn Ser Leu Val Ile

50 55 60

Tyr Val Ile Cys Arg His Lys His Met Gln Thr Val Thr Asn Phe Tyr

65 70 75 80

Ile Ala Asn Leu Ala Ala Thr Asp Val Thr Phe Leu Leu Cys Cys Val

85 90 95

Pro Phe Thr Ala Leu Leu Tyr Pro Leu Pro Ala Trp Val Leu Gly Asp

100 105 110

Phe Met Cys Lys Phe Val Asn Tyr Ile Gln Gln Val Ser Val Gln Ala
115 120 125
Thr Cys Ala Thr Leu Thr Ala Met Ser Val Asp Arg Trp Tyr Val Thr
130 135 140
Val Phe Pro Leu Arg Ala Leu His Arg Arg Thr Pro Arg Leu Ala Leu
145 150 155 160
Ala Val Ser Leu Ser Ile Trp Val Gly Ser Ala Ala Val Ser Ala Pro
165 170 175
Val Leu Ala Leu His Arg Leu Ser Pro Gly Pro Arg Thr Tyr Cys Ser
180 185 190
Glu Ala Phe Pro Ser Arg Ala Leu Glu Arg Ala Phe Ala Leu Tyr Asn
195 200 205
Leu Leu Ala Leu Tyr Leu Leu Pro Leu Leu Ala Thr Cys Ala Cys Tyr
210 215 220
Gly Ala Met Leu Arg His Leu Gly Arg Ala Ala Val Arg Pro Ala Pro
225 230 235 240
Thr Asp Gly Ala Leu Gln Gly Gln Leu Leu Ala Gln Arg Ala Gly Ala
245 250 255
Val Arg Thr Lys Val Ser Arg Leu Val Ala Ala Val Val Leu Leu Phe
260 265 270
Ala Ala Cys Trp Gly Pro Ile Gln Leu Phe Leu Val Leu Gln Ala Leu
275 280 285
Gly Pro Ser Gly Ala Trp His Pro Arg Ser Tyr Ala Ala Tyr Ala Val
290 295 300
Lys Ile Trp Ala His Cys Met Ser Tyr Ser Asn Ser Ala Leu Asn Pro
305 310 315 320
Leu Leu Tyr Ala Phe Leu Gly Ser His Phe Arg Gln Ala Phe Cys Arg
325 330 335
Val Cys Pro Cys Cys Arg Gln Arg Gln Arg Arg Pro His Thr Ser Ala

340 345 350

His Ser Asp Arg Ala Ala Thr His Thr Val Pro His Ser Arg Ala Ala

355 360 365

His Pro Val Arg Ile Arg Ser Pro Glu Pro Gly Asn Pro Val Val Arg

370 375 380

Ser Pro Cys Ala Gln Ser Glu Arg Thr Ala Ser Leu

385 390 395

<210> 25

<211> 1188

<212> DNA

<213> Mouse

<400> 25

atggccaccg aggcgacatt ggctcccaat gtgacctggt gggctccgtc caacgcttca 60

ggatgcccag gctgcggtgt caacgcctcg gatgacccag gctctgcgcc aaggcccctg 120

gatgcctggc tggttcccct gtttttcgct acactcatgt tgcttgggct ggtcggaaac 180

tcattggtca tctacgttat ctgccgccac aagcacatgc agacagttac caacttctac 240

atcgctaacc tggctgccac agacgtcact ttcctactgt gctgcgtgcc cttcaccgca 300

ctcctctacc cgctgcccgc ctgggtgctg ggagacttca tgtgcaaatt cgtcaactac 360

atccagcagg tctcggtgca agccacatgt gccactctga cggccatgag tgtggaccgc 420

tggtatgtga ctgtgttccc gctgcgtgca cttcaccgcc gcactccgcg cctggccctg 480

gctgtcagcc tcagcatctg ggtggggtca gcagctgtgt ccgccccggt gctggccctg 540

caccgcctgt cgccagggcc tcgcacctac tgcagcgagg cgtttcccag ccgcgccctg 600

gagcgcgcct tcgcgctcta caacctgctg gctctatatc tgctgccgct gctcgccacc 660

tgcgcctgct acggcgccat gctgcgccac ctgggccgtg cggctgtacg ccccgcaccc 720

actgacggcg ccctgcaggg acagctgcta gcacagcgcg ccggagcagt gcgcaccaag 780

gtctcccggc tggtggccgc tgtcgtcctg ctcttcgccg cctgctgggg cccgatccag 840

ctgttcctgg tgcttcaagc cctgggcccc tcgggggcct ggcaccctcg aagctatgcc 900

gcctacgcgg tcaagatctg ggctcactgc atgtcctaca gcaactcggc gctcaatccg 960

ctgctctatg ccttcctggg ttcacacttc agacaggcct tctgccgcgt gtgcccctgc 1020

tgccggcaac gccagcgccg gccccacacg tcagcgcact cggaccgagc tgcaactcac 1080

actgtgccgc acagccgtgc tgcgcaccct gtgcggatca ggagcccgga gcctgggaac 1140

cctgtggtgc gctcgccctg cgctcagagt gaacgcactg cctcactc 1188

<210> 26

<211> 26

<212> DNA

<213> Artificial Sequence

<220>

<223> Primer

<400> 26

tccccacagt cccaggacac aatcct 26

<210> 27

<211> 26

<212> DNA

<213> Artificial Sequence

<220>

<223> Primer

<400> 27

caccagcgca agcagcctgg gatgct 26